# EUROPEAN PATENT APPLICATION

(11) **EP 1 902 729 A1**
(43) Date of publication of application: **26.03.2008**
(21) Application number: 06746434.7
(22) Date of filing: 16.05.2006
(51) Int. Cl.: A61K 45/00, A01K 67/027, A61K 31/138, A61K 31/565, A61K 31/57, A61K 31/7088, A61K 39/395, A61K 48/00, A61P 1/16, A61P 1/18, A61P 9/10, A61P 17/00, A61P 21/00, A61P 25/28, A61P 35/00, A61P 43/00, C12N 15/09, C12Q 1/02, C12Q 1/68, G01N 33/15, G01N 33/50

(54) **Int6 PROTEIN INVOLVED IN HYPOXIA STRESS INDUCTION AND USE THEREOF**

(30) Priority: 16.05.2005 JP 2005142553
(71) Applicant: Tokyo Metropolitan Organization for Medical Research, Shinjuku-ku, Tokyo 163-8001 (JP); Caretis Co. , Ltd., Mobara-shi Chiba 2970012 (JP)
(72) Inventor: SHIBASAKI, Futoshi, 1750094 (JP); CHEN, Li, 1130031 (JP); SAKATA, Kazuhiko, 2970012 (JP)
(74) Representative: Bohmann, Armin K.
(86) International application number: PCT/JP2006/309720
(87) International publication number: WO 2006/123644

(57) **Abstract**

The present inventors successfully identified an HIF2α binding factor, Int6, which has binding activity to the HIF2α, as a factor involved in hypoxic stress response that promotes angiogenesis. Furthermore, the present inventors discovered that Int6 suppresses HIF2α-mediated transcription of hypoxic stress-responsive genes by interacting with HIF2α. Substances that inhibit Int6 expression or its function promote angiogenesis by enhancing the activity of HIF2α to promote transcription of hypoxic stress-responsive genes, and are expected to have therapeutic effects for various vascular diseases including myocardial infarction.

SEQUENCE LISTING

<110> Tokyo Metropolitan Organization for Medical Research
Caretis, Co., Ltd.

<120> Int6 protein involved in hypoxia stress induction and use thereof

<130> T 10011 EP

<140> EP 06746434.7
<141> 2006-05-16

<150> JP 2005-142553
<151> 2005-05-16

<160> 11

<170> PatentIn version 3.1

<210> 1
<211> 1510
<212> DNA
<213> Homo sapiens

<400> 1
ggactccctt ttctttggca agatggcgga gtacgacttg actactcgca tcgcgcactt 60

tttggatcgg catctagtct ttccgcttct tgaatttctc tctgtaaagg agatatataa 120

tgaaaaggaa ttattacaag gtaaattgga ccttcttagt gataccaaca tggtagactt 180

tgctatggat gtatacaaaa acctttattc tgatgatatt cctcatgctt tgagagagaa 240

aagaaccaca gtggttgcac aactgaaaca gcttcaggca gaaacagaac caattgtgaa 300

gatgtttgaa gatccagaaa ctacaaggca aatgcagtca accagggatg gtaggatgct 360

ctttgactac ctggcggaca agcatggttt taggcaggaa tatttagata cactctacag 420

atatgcaaaa ttccagtacg aatgtgggaa ttactcagga gcagcagaat atctttattt 480

ttttagagtg ctggttccag caacagatag aaatgcttta agttcactct ggggaaagct 540

ggcctctgaa atcttaatgc agaattggga tgcagccatg gaagacctta cacggttaaa 600

agagaccata gataataatt ctgtgagttc tccacttcag tctcttcagc agagaacatg 660

gctcattcac tggtctctgt ttgttttctt caatcacccc aaaggtcgcg ataatattat 720

tgacctcttc ctttatcagc cacaatatct taatgcaatt cagacaatgt gtccacacat 780

tcttcgctat ttgactacag cagtcataac aaacaaggat gttcgaaaac gtcggcaggt 840

tctaaaagat ctagttaaag ttattcaaca ggagtcttac acatataaag acccaattac 900

agaatttgtt gaatgtttat atgttaactt tgactttgat ggggctcaga aaaagctgag 960

ggaatgtgaa tcagtgcttg tgaatgactt cttcttggtg gcttgtcttg aggatttcat 1020

tgaaaatgcc cgtctcttca tatttgagac tttctgtcgc atccaccagt gtatcagcat 1080

taacatgttg gcagataaat tgaacatgac tccagaagaa gctgaaaggt ggattgtaaa 1140

tttgattaga aatgcaagac tggatgccaa gattgattct aaattaggtc atgtggttat 1200

gggtaacaat gcagtctcac cctatcagca agtgattgaa aagaccaaaa gcctttcctt 1260

tagaagccag atgttggcca tgaatattga gaagaaactt aatcagaata gcaggtcaga 1320

ggctcctaac tgggcaactc aagattctgg cttctactga agaaccataa agaaaagatg 1380

aaaaaaaaaa ctatcaaaga aagatgaaat aataaaacta ttatataaag ggtgacttac 1440

attttggaaa caacatatta cgtataaatt ttgaagaatt ggaataaaat tgattcattt 1500

taaaaaaaaa 1510

<210> 2
<211> 445
<212> PRT
<213> Homo sapiens

<400> 2

Met Ala Glu Tyr Asp Leu Thr Thr Arg Ile Ala His Phe Leu Asp Arg
1               5                   10                  15

His Leu Val Phe Pro Leu Leu Glu Phe Leu Ser Val Lys Glu Ile Tyr
            20                  25                  30

Asn Glu Lys Glu Leu Leu Gln Gly Lys Leu Asp Leu Leu Ser Asp Thr
        35                  40                  45

Asn Met Val Asp Phe Ala Met Asp Val Tyr Lys Asn Leu Tyr Ser Asp
    50                  55                  60

Asp Ile Pro His Ala Leu Arg Glu Lys Arg Thr Thr Val Val Ala Gln
65                  70                  75                  80

Leu Lys Gln Leu Gln Ala Glu Thr Glu Pro Ile Val Lys Met Phe Glu
                85                  90                  95

Asp Pro Glu Thr Thr Arg Gln Met Gln Ser Thr Arg Asp Gly Arg Met
            100                 105                 110

Leu Phe Asp Tyr Leu Ala Asp Lys His Gly Phe Arg Gln Glu Tyr Leu
        115                 120                 125

Asp Thr Leu Tyr Arg Tyr Ala Lys Phe Gln Tyr Glu Cys Gly Asn Tyr
    130                 135                 140

Ser Gly Ala Ala Glu Tyr Leu Tyr Phe Phe Arg Val Leu Val Pro Ala
145                 150                 155                 160

Thr Asp Arg Asn Ala Leu Ser Ser Leu Trp Gly Lys Leu Ala Ser Glu

                165                 170                 175

Ile Leu Met Gln Asn Trp Asp Ala Ala Met Glu Asp Leu Thr Arg Leu
            180                 185                 190

Lys Glu Thr Ile Asp Asn Asn Ser Val Ser Ser Pro Leu Gln Ser Leu
        195                 200                 205

Gln Gln Arg Thr Trp Leu Ile His Trp Ser Leu Phe Val Phe Phe Asn
    210                 215                 220

His Pro Lys Gly Arg Asp Asn Ile Ile Asp Leu Phe Leu Tyr Gln Pro
225                 230                 235                 240

Gln Tyr Leu Asn Ala Ile Gln Thr Met Cys Pro His Ile Leu Arg Tyr
                245                 250                 255

Leu Thr Thr Ala Val Ile Thr Asn Lys Asp Val Arg Lys Arg Arg Gln
            260                 265                 270

Val Leu Lys Asp Leu Val Lys Val Ile Gln Gln Glu Ser Tyr Thr Tyr
        275                 280                 285

Lys Asp Pro Ile Thr Glu Phe Val Glu Cys Leu Tyr Val Asn Phe Asp
    290                 295                 300

Phe Asp Gly Ala Gln Lys Lys Leu Arg Glu Cys Glu Ser Val Leu Val
305                 310                 315                 320

Asn Asp Phe Phe Leu Val Ala Cys Leu Glu Asp Phe Ile Glu Asn Ala
                325                 330                 335

Arg Leu Phe Ile Phe Glu Thr Phe Cys Arg Ile His Gln Cys Ile Ser
            340                 345                 350

Ile Asn Met Leu Ala Asp Lys Leu Asn Met Thr Pro Glu Glu Ala Glu
        355                 360                 365

Arg Trp Ile Val Asn Leu Ile Arg Asn Ala Arg Leu Asp Ala Lys Ile
    370                 375                 380

Asp Ser Lys Leu Gly His Val Val Met Gly Asn Asn Ala Val Ser Pro
385                 390                 395                 400

Tyr Gln Gln Val Ile Glu Lys Thr Lys Ser Leu Ser Phe Arg Ser Gln
                405                 410                 415

Met Leu Ala Met Asn Ile Glu Lys Lys Leu Asn Gln Asn Ser Arg Ser
            420                 425                 430

Glu Ala Pro Asn Trp Ala Thr Gln Asp Ser Gly Phe Tyr
        435                 440                 445

<210>  3
<211>  5186
<212>  DNA
<213>  Homo sapiens

<400>  3
gccacacggg tccggtgccc gctgcgcttc cgccccagcg ctcctgaggc ggccgtacaa      60

tcctcggcag tgtcctgaga ctgtatggtc agctcagccc ggcctccgac tccttccgac     120

tcccagcatt cgagccactt ttttttttct ttgaaaactc agaaaagtga ctccttttcc     180

agggaaaaag gaacttgggt tcccttctct ccgtcctctt ttcgggtctg acagcctcca     240

cccactcctt ccccggaccc cgcctccgcg cgcaggttcc tcccagtcac ctttctccac     300

ccccgccccc gcacctagcc cgccgcgcgc caccttccac ctgactgcgc ggggcgctcg     360

ggacctgcgc gcacctcgga ccttcaccac ccgcccgggc cgcggggagc ggacgagggc     420

cacagccccc cacccgccag ggagcccagg tgctcggcgt ctgaacgtct caaagggcca     480

cagcgacaat gacagctgac aaggagaaga aaaggagtag ctcggagagg aggaaggaga     540

agtcccggga tgctgcgcgg tgccggcgga gcaaggagac ggaggtgttc tatgagctgg     600

cccatgagct gcctctgccc cacagtgtga gctcccatct ggacaaggcc tccatcatgc     660

gactggcaat cagcttcctg cgaacacaca agctcctctc ctcagtttgc tctgaaaacg     720

agtccgaagc cgaagctgac cagcagatgg acaacttgta cctgaaagcc ttggagggtt     780

tcattgccgt ggtgacccaa gatggcgaca tgatctttct gtcagaaaac atcagcaagt     840

tcatgggact tacacaggtg gagctaacag gacatagtat ctttgacttc actcatccct     900

gcgaccatga ggagattcgt gagaacctga gtctcaaaaa tggctctggt tttgggaaaa     960

aaagcaaaga catgtccaca gagcgggact tcttcatgag gatgaagtgc acggtcacca    1020

acagaggccg tactgtcaac ctcaagtcag ccacctggaa ggtcttgcac tgcacgggcc    1080

aggtgaaagt ctacaacaac tgccctcctc acaatagtct gtgtggctac aaggagcccc    1140

tgctgtcctg cctcatcatc atgtgtgaac caatccagca cccatcccac atggacatcc    1200

ccctggatag caagaccttc ctgagccgcc acagcatgga catgaagttc acctactgtg    1260

atgacagaat cacagaactg attggttacc accctgagga gctgcttggc cgctcagcct    1320

atgaattcta ccatgcgcta gactccgaga acatgaccaa gagtcaccag aacttgtgca    1380

ccaagggtca ggtagtaagt ggccagtacc ggatgctcgc aaagcatggg ggctacgtgt    1440

ggctggagac ccaggggacg gtcatctaca accctcgcaa cctgcagccc cagtgcatca    1500

tgtgtgtcaa ctacgtcctg agtgagattg agaagaatga cgtggtgttc tccatggacc 1560
agactgaatc cctgttcaag ccccacctga tggccatgaa cagcatcttt gatagcagtg 1620
gcaagggggc tgtgtctgag aagagtaact tcctattcac caagctaaag gaggagcccg 1680
aggagctggc ccagctggct cccaccccag gagacgccat catctctctg gatttcggga 1740
atcagaactt cgaggagtcc tcagcctatg gcaaggccat cctgcccccg agccagccat 1800
gggccacgga gttgaggagc cacagcaccc agagcgaggc tgggagcctg cctgccttca 1860
ccgtgcccca ggcagctgcc ccgggcagca ccacccccag tgccaccagc agcagcagca 1920
gctgctccac gcccaatagc cctgaagact attacacatc tttggataac gacctgaaga 1980
ttgaagtgat tgagaagctc ttcgccatgg acacagaggc caaggaccaa tgcagtaccc 2040
agacggattt caatgagctg gacttggaga cactggcacc ctatatcccc atggacgggg 2100
aagacttcca gctaagcccc atctgccccg aggagcggct cttggcggag aacccacagt 2160
ccacccccca gcactgcttc agtgccatga caaacatctt ccagccactg gcccctgtag 2220
ccccgcacag tcccttcctc ctggacaagt ttcagcagca gctggagagc aagaagacag 2280
agcccgagca ccggcccatg tcctccatct tctttgatgc cggaagcaaa gcatccctgc 2340
caccgtgctg tggccaggcc agcacccctc tctcttccat gggggcagaa tccaataccc 2400
agtggccccc agatccacca ttacattttg ggcccacaaa gtgggccgtc ggggatcagc 2460
gcacagagtt cttgggagca gcgccgttgg ggccccctgt ctctccaccc catgtctcca 2520
ccttcaagac aaggtctgca aagggttttg gggctcgagg cccagacgtg ctgagtccgg 2580
ccatggtagc cctctccaac aagctgaagc tgaagcgaca gctggagtat gaagagcaag 2640
ccttccagga cctgagcggg ggggacccac ctggtggcag cacctcacat ttgatgtgga 2700
aacggatgaa gaacctcagg ggtgggagct gccctttgat gccggacaag ccactgagcg 2760
caaatgtacc caatgataag ttcacccaaa accccatgag gggcctgggc catcccctga 2820
gacatctgcc gctgccacag cctccatctg ccatcagtcc cggggagaac agcaagagca 2880
ggttcccccc acagtgctac gccacccagt accaggacta cagcctgtcg tcagcccaca 2940
aggtgtcagg catggcaagc cggctgctcg ggccctcatt tgagtcctac ctgctgcccg 3000
aactgaccag atatgactgt gaggtgaacg tgcccgtgct gggaagctcc acgctcctgc 3060
aaggagggga cctcctcaga gccctggacc aggccacctg agccaggcct tctacctggg 3120
cagcacctct gccgacgccg tcccaccagc ttcactctct ccgtctgttt ttgcaactag 3180
gtatttctaa cgccagcaca ctatttacaa gatggactta cctggcagac ttgcccaggt 3240
caccaagcag tggccttttt ctgagatgct cactttatta tccctatttt taaagtacac 3300
aattgtttta cctgttctga aatgttctta aattttgtag gatttttttc ctccccacct 3360

tcaatgactt ctaatttata ttatccatag gtttctctcc ctccttctcc ttctcacaca 3420

caactgtcca tactaacaag tttggtgcat gtctgttctt ctgtagggag aagctttagc 3480

ttcattttac taaaaagatt cctcgttatt gttgttgcca aagagaaaca aaaatgattt 3540

tgctttccaa gcttggtttg tggcgtctcc ctcgcagagc ccttctcgtt tcttttttaa 3600

actaatcacc atattgtaaa tttcagggtt tttttttttt tgtttaagct gactctttgc 3660

tctaattttg gaaaaaaaga aatgtgaagg gtcaactcca acgtatgtgg ttatctgtga 3720

aagttgcaca gcgtggcttt tcctaaactg gtgtttttcc cccgcatttg gtggattttt 3780

tattattatt caaaaacata actgagtttt ttaaaagagg agaaaattta tatctgggtt 3840

aagtgtttat catatatatg ggtactttgt aatatctaaa aacttagaaa cggaaatgga 3900

atcctgctca caaaatcact ttaagatctt ttcgaagctg ttaatttttc ttagtgttgt 3960

ggacactgca gacttgtcca gtgctcccac ggcctgtacg gacactgtgg aaggcctccc 4020

tctgtcggct ttttgccatc tgtgatatgc cataggtgtg acaatccgag cagtggagtc 4080

attcagcggg agcactgcgc gctatcccct cacattctct atgtactatg tatgtatgta 4140

ttattattat tgctgccaag agggtctgat ggcacgttgt ggggtcgggg ggtggggcgg 4200

ggaagtgctc taacttttct taaggttttg ttgctagccc ttcaagtgca ctgagctatg 4260

tgactcggat ggtctttcac acggcacatt tggacatttc cagaactacc atgagatggt 4320

ttagacggga attcatgcaa atgaggggtc aaaaatggta tagtgacccc gtccacgtcc 4380

tccaagctca cgaccttgga gccccgtgga gctggactga ggaggaggct gcacagcggg 4440

agagcagctg gtccagacca gccctgcagc ccccactcag ccggcagcca gatggccccg 4500

caaggcctcc agggatggcc cctagccaca ggccctggct gaggtctctg ggtcggtcag 4560

tgacatgtag gtaggaagca ctgaaaatag tgttcccaga gcactttgca actccctggg 4620

taagagggac gacacctctg gtttttcaat accaattaca tggaactttt ctgtaatggg 4680

tacaatgaag aagtttctaa aaacacacac aaagcacatt gggccaacta tttagtaagc 4740

ccggatagac ttattgccaa aaacaaaaaa tagctttcaa aagaaattta agttctatga 4800

gaaattcctt agtcatggtg ttgcgtaaat catattttag ctgcacggca ttaccccaca 4860

cagggtggca gaacttgaag ggttactgac gtgtaaatgc tggtatttga tttcctgtgt 4920

gtgttgccct ggcattaagg gcattttacc cttgcagttt tactaaaaca ctgaaaaata 4980

ttccaagctt catattaacc ctacctgtca acgtaacgat ttcatgaacg ttattatatt 5040

gtcgaattcc tactgacaac attataactg tatgggagct taactttata aggaaatgta 5100

ttttgacact ggtatcttat taaagtattc tgatcctaaa aaaaaaaaaa aaaaaaaaaa 5160

aaaaaaaaaa aaaaaaaaaa aaaaaa 5186

<210>  4
<211>  870
<212>  PRT
<213>  Homo sapiens

<400>  4

<210>  5
<211>  64
<212>  DNA
<213>  Artificial

<220>
<223>  an artificially synthesized sequence

<400>  5
gatcccaaga accacagtgg ttgcattcaa gagatgcaac cactgtggtt cttttttttg      60

gaaa                                                                    64

<210>  6
<211>  64
<212>  DNA
<213>  Artificial

<220>
<223>  an artificially synthesized sequence

<400>  6
gatcccaagc atggttttag gcaggttcaa gagacctgcc taaaaccatg cttttttttg      60

gaaa                                                                    64

<210>  7
<211>  64
<212>  DNA
<213>  Artificial

<220>
<223>  an artificially synthesized sequence

<400>  7
gatcccaatg ctttaagttc actctttcaa gagaagagtg aacttaaagc attttttttg      60

gaaa                                                                    64

<210>  8
<211>  64
<212>  DNA
<213>  Artificial

<220>
<223>  an artificially synthesized sequence

<400>  8
   gatcccaaca tggtagactt tgctattcaa gagatagcaa agtctaccat gttttttttg      60
   gaaa                                                                    64

<210>  9
<211>  53
<212>  DNA
<213>  Mus musculus

<400>  9
aatatggtgg actttgctat tcaagagata gcaaagtcca ccatattttt ttt            53

<210>  10
<211>  53
<212>  DNA
<213>  Mus musculus

<400>  10
aagaaccaca gttgttgcgt tcaagagacg caacaactgt ggttcttttt ttt            53

<210>  11
<211>  53
<212>  DNA
<213>  Mus musculus

<400>  11
aaacatgggt ttaggcaagt tcaagagact tgcctaaacc catgtttttt ttt            53

## Description

### Technical Field

The present invention relates to the Int6 protein which regulates hypoxic stress response, therapeutic agents for vascular diseases and anticancer agents comprising substances that regulate the function or expression of the protein, and methods of screening for these pharmaceutical agents.

### Background Art

Organisms have maintained their life by adapting to various environmental changes. Responsive reactions to such environmental stress take place not only at the physiological level but also at the cellular level, and stress response mechanisms at the molecular level are being elucidated. In particular, the presence of oxygen is one of the most important environmental factors for all organisms living on earth.

Since oxygen is an energy source necessary for continued cellular function in organisms, lack of oxygen is a serious problem affecting the continuation of life. Causes that put organisms under hypoxic conditions include anemia, abnormal cardiopulmonary function, and high-altitude life. Under such conditions, each cell in a living body changes the expression patterns of various genes and tries to adapt to hypoxic stress. For example, glycolytic pathways are activated and angiogenesis is induced as responsive reactions to cellular hypoxic stress.

Some of the genes involved in hypoxic stress response have a hypoxia response element (HRE) in their upstream region. A hypoxia inducible transcription factor (HIF) binds to HRE and promotes transcription. HIFs have been analyzed in detail, and are known to produce vascular endothelial growth factors (VEGFs) which are necessary for angiogenesis in tumors, and to regulate transcription of many glycolytic pathway enzymes involved in energy metabolism in mitochondria. In solid tumors (for example, cancers), the condition becomes fairly hypoxic inside the tumor in spite of abundant blood flow, and this condition consequently promotes angiogenesis. Besides angiogenesis, HIFs take on various roles in cells and are reported to be involved in energy metabolism pathways, erythrocyte growth, iron metabolism, cell growth, cell death, dopamine metabolism, and such. For example, enhancement of erythropoietin gene expression is necessary for erythrocyte growth under hypoxia, and increased expression of VEGF genes is important for angiogenesis. Transcription factors including hypoxia inducible factor 1α (HIF1α), HIF 1-like factor (HLF), and GATA-2 are known to play important roles in regulating the expression of these hypoxia-responsive genes. HIF1α is deeply involved in cellular responses to hypoxia. It is degraded by the ubiquitin-proteasome system at normal oxygen concentration (21 %), but under hypoxic conditions it escapes this degradation and transfers to the nucleus to evoke induction of various factors as a transcription factor.

pVHL is an example of a factor that degrades HIF1α at normal oxygen concentration. pVHL binds to and ubiquitinates HIF1α for degradation. At this time, proline hydroxylase (PHD) hydroxylates the proline residues on HIF1α, and this enables pVHL to recognize this site.

HIFs are mainly classified into three types, HIF1α, HIF2α, and HIF3α. In contrast to HIF1α which is expressed ubiquitously, HIF2α is expressed specifically in vascular endothelial cells, skeletal muscles, cardiac muscles, and such, and HIF3α is expressed specifically in the cerebral nervous system. HIF1α is known to be related to ischemic diseases such as cerebral infarction or myocardial infarction (see Non-Patent Documents 1 to 4). HIF2α is involved in angiogenesis in hypoxic stress-related diseases, particularly in cancers (see Non-Patent Document 5). Although several factors that bind to this HIF2α have been reported, factors that regulate its function have not been found.
Non-Patent Document 1: Semenza G L., "HIF-1 and mechanisms of hypoxia sensing" Curr. Opin. Cell Biol., 2001 Apr, Vol. 13(2), p.167-71.
Non-Patent Document 2: Giaccia A. and other two authors, "HIF-1 as a target for drug development" 2003 Oct, Vol. 2(10), p.803-11.
Non-Patent Document 3: Jelkmann W., "Effects of erythropoietin on brain function" Curr. Pharm. Biotechnol., 2005 Feb, Vol. 6(1), p.65-79.
Non-Patent Document 4: Marti H. H., "Erythropoietin and the hypoxic brain" J. Exp. Biol., 2004 Aug, Vol. 207(Pt 18), p.3233-42.
Non-Patent Document 5: Favier J. and other three authors, "Coexpression of endothelial PAS protein 1 with essential angiogenic factors suggests its involvement in human vascular development" Dev. Dyn., 2001 Nov, Vol. 222(3), p.377-88.

### Disclosure of the Invention

### Problems to Be Solved by the Invention

The present invention was achieved in view of the above circumstances. An objective of the present invention is to elucidate mechanisms of angiogenesis-related gene expression response under hypoxic stress, and a further objective is to identify factors that regulate the HIF2α function of, in particular, promoting hypoxic stress response that induces angiogenesis. More specifically, an objective is to provide pharmaceutical agents that regulate hypoxic stress response, pharmaceutical agents having therapeutic effects on vascular diseases, and methods of screening for these pharmaceutical agents.

### Means for Solving the Problems

The present inventors have conducted dedicated research to solve the above-mentioned objectives, and succeeded in newly identifying Int6, which has binding activity to HIF2α, as a factor involved in angiogenesis-promoting hypoxic stress response.

Transcription of hypoxia-responsive genes is known to be promoted by the action of HIF2α under hypoxic stress. The present invention revealed that under such conditions, the transcriptional function of HIF2α is regulated by Int6. More specifically, the present invention revealed that Int6 interacts with HIF2α to suppress the transcription of hypoxic stress-responsive genes by HIF2α.

Substances that inhibit Int6 gene expression or its function (for example, binding activity to HIF2α) are expected to increase angiogenic effects by enhancing the activity of HIF2α to promote the transcription of hypoxic stress-responsive genes. These substances are expected to have significant angiogenic effects and therapeutic effects on various vascular diseases such as arteriosclerosis obliterans, restenosis after percutaneous vasodilation treatment (PCT), and myocardial infarction.

Furthermore, the present inventors prepared laboratory animals whose Int6 gene expression is suppressed by siRNA, and found that angiogenic effects are actually increased in those laboratory animals. Therefore, the present inventors have successfully confirmed that at the laboratory animal level, substances inhibiting Int6 expression or its function actually increase angiogenic effects effectively. In particular, siRNAs that are expected to have specific suppressive effects on Int6 gene expression promote the expression of genes that have therapeutic effects for vascular diseases, by suppressing the expression of transcription regulatory factors, and are highly expected to be an entirely novel-concept therapeutic agent for vascular diseases.

Furthermore, substances that enhance Int6 expression or its function are expected to suppress angiogenic effects by decreasing the activity of HIF2α to promote transcription of hypoxic stress-responsive genes. Since angiogenic effects are increased in cancer tissues, these substances may become novel anticancer agents whose mechanism involves suppressing angiogenesis.

Furthermore, the present inventors were the first to discover that female hormones suppress the expression of Int6. That is, female hormones enhance the activity of HIF2α to promote the transcription of hypoxic stress-responsive genes by suppressing Int6 gene expression, and are considered to promote angiogenesis as a result. Therefore, female hormones are effective for treating, for example, diseases in which the pathologies may be improved by improving blood flow volume (for example, arteriosclerosis obliterans). In contrast, female hormone-suppressing agents are expected to have effects such as anticancer effects through suppression of angiogenesis.

Based on these findings made by the present inventors, therapeutic agents for vascular diseases, anticancer agents, or such can be screened.

The present invention relates to pharmaceutical agents that regulate hypoxic stress response, pharmaceutical agents having therapeutic effects against vascular diseases or cancers, and methods of screening for these pharmaceutical agents. More specifically, the present invention provides:
[1] a hypoxic stress response-promoting agent comprising as an active ingredient a substance that suppresses Int6 protein expression or a substance that suppresses Int6 protein function;
[2] the hypoxic stress response-promoting agent of [1], wherein the substance that suppresses Int6 protein expression is a compound selected from the group consisting of:
   (a) an antisense nucleic acid against an Int6 gene transcript or a part thereof;
   (b) a nucleic acid having a ribozyme activity of specifically cleaving an Int6 gene transcript; and
   (c) a nucleic acid having a function of inhibiting Int6 gene expression by RNAi effect;
[3] the hypoxic stress response-promoting agent of [1], wherein the substance that suppresses Int6 protein expression is a female hormone, a female hormone secretion-promoting agent, or a receptor agonist of female hormone;
[4] the hypoxic stress response-promoting agent of [1], wherein the substance that suppresses Int6 protein function is a compound selected from the group consisting of:
   (a) an antibody that binds to an Int6 protein;
   (b) a low-molecular-weight compound that binds to an Int6 protein; and
   (c) a compound that inhibits the binding activity between Int6 protein and HIF2α protein;
[5] the hypoxic stress response-promoting agent of any one of [1] to [4], wherein the promotion of hypoxic stress response is induction of angiogenesis;
[6] a hypoxic stress response-suppressing agent, which comprises as an active ingredient a substance that enhances Int6 protein expression or a substance that enhances Int6 protein function;
[7] the hypoxic stress response-suppressing agent of [6], wherein the substance that enhances Int6 protein expression is a female hormone-suppressing agent;
[8] the hypoxic stress response-suppressing agent of [7], wherein the suppression of hypoxic stress response is suppression of angiogenesis;
[9] a therapeutic agent for a vascular disease, which comprises the hypoxic stress response-promoting agent of any one of [1] to [5] as an active ingredient;
[10] the therapeutic agent for a vascular disease of [9], wherein the vascular disease is an ischemic brain or heart disease, a neurodegenerative disease, a traumatic brain injury, or a hepatic, pancreatic, muscular, or skin disease;
[11] a therapeutic agent for an angiogenesis-related disease (for example, an anticancer agent), which comprises the hypoxic stress response-suppressing agent of any one of [6] to [8] as an active ingredient;
[12] the therapeutic agent for an angiogenesis-related disease of [11], wherein the angiogenesis-related disease is a cancer disease;
[13] a non-human transgenic animal whose Int6 gene expression is artificially suppressed;
[14] the non-human transgenic animal of [13], wherein the Int6 gene expression is suppressed by the action of any one of the nucleic acids of:
   (a) an antisense nucleic acid against an Int6 gene transcript or a part thereof;
   (b) a nucleic acid having a ribozyme activity of specifically cleaving an Int6 gene transcript; and
   (c) a nucleic acid having a function of inhibiting Int6 gene expression by RNAi effect;
[15] the non-human transgenic animal of [13] or [14], wherein angiogenic effect is promoted;
[16] a method of screening for a therapeutic agent for vascular disease, wherein a compound that decreases the level of Int6 gene expression or Int6 protein activity is selected;
[17] a method of screening for a therapeutic agent for vascular disease, which comprises the steps of:
   (a) contacting a test compound with Int6 gene-expressing cells;
   (b) measuring the level of Int6 gene expression in said cells; and
   (c) selecting a compound that decreases the expression level compared with that measured in the absence of the test compound;
[18] a method of screening for a therapeutic agent for vascular disease, which comprises the steps of:
   (a) contacting a test compound with cells or an extract solution of cells comprising a DNA which comprises a structure in which a reporter gene is operably linked to the transcriptional regulatory region of an Int6 gene;
   (b) measuring the expression level of said reporter gene; and
   (c) selecting a compound that decreases the expression level compared with that measured in the absence of the test compound;
[19] a method of screening for a therapeutic agent for vascular disease, which comprises the steps of:
   (a) contacting a test compound with an Int6 protein, or cells or an extract solution of cells expressing the protein;
   (b) measuring the activity of said protein; and
   (c) selecting a compound that decreases the activity of said protein compared with that measured in the absence of the test compound;
[20] a method of screening for a therapeutic agent for vascular disease, which comprises selecting a compound that suppresses the binding activity between Int6 protein and HIF2α protein;
[21] a method of screening for a therapeutic agent for vascular disease, which comprises the steps of:
   (a) contacting a test compound with an Int6 protein and an HIF2α protein;
   (b) measuring the binding activity between the Int6 protein and the HIF2α protein; and
   (c) selecting a compound that decreases said binding activity compared with that measured in the absence of the test compound;
[22] a method of screening for a therapeutic agent for angiogenesis-related disease (for example, an anticancer agent), which comprises selecting a compound that increases the level of Int6 gene expression or Int6 protein activity;
[23] a method of screening for a therapeutic agent for angiogenesis-related disease (for example, an anticancer agent), which comprises the steps of:
   (a) contacting a test compound with Int6 gene-expressing cells;
   (b) measuring the level of Int6 gene expression in said cells; and
   (c) selecting a compound that increases the expression level compared with that measured in the absence of the test compound;
[24] a method of screening for a therapeutic agent for angiogenesis-related disease (for example, an anticancer agent), which comprises the steps of:
   (a) contacting a test compound with cells or an extract solution of cells comprising a DNA which comprises a structure in which a reporter gene is operably linked to the transcriptional regulatory region of an Int6 gene;
   (b) measuring the expression level of said reporter gene; and
   (c) selecting a compound that increases the expression level compared with that measured in the absence of the test compound;
[25] a method of screening for a therapeutic agent for angiogenesis-related disease (for example, an anticancer agent), which comprises the steps of:
   (a) contacting a test compound with an Int6 protein, or cells or an extract solution of cells expressing the protein;
   (b) measuring the activity of said protein; and
   (c) selecting a compound that increases the activity of said protein compared with that measured in the absence of the test compound;
[26] a method of screening for a therapeutic agent for angiogenesis-related disease (for example, an anticancer agent), which comprises selecting a compound that enhances the binding activity between Int6 protein and HIF2α protein;
[27] a method of screening for a therapeutic agent for angiogenesis-related disease (for example, an anticancer agent), which comprises the steps of:
   (a) contacting a test compound with an Int6 protein and an HIF2α protein;
   (b) measuring the binding activity between the Int6 protein and the HIF2α protein; and
   (c) selecting a compound that increases said binding activity compared with that measured in the absence of the test compound; and
[28] a method of screening for a therapeutic agent for angiogenesis-related disease (for example, an anticancer agent), which comprises the steps of:
   (a) administering a test compound to the non-human transgenic animal of any one of [13] to [15];
   (b) measuring the level of Int6 expression or activity in said non-human transgenic animal, or the angiogenic effect in said animal; and
   (c) selecting a compound that decreases the angiogenic effect, or a compound that increases the level of Int6 expression or activity compared with that when the test compound is not administered (control).

   Furthermore, the present invention provides:
[29] an angiogenesis-inducing agent comprising a dominant negative Int6 mutant as an active ingredient;
[30] the angiogenesis-inducing agent of [29], wherein the dominant negative Int6 mutant is an Int6 protein mutant in which the C terminal PINT region of the Int6 protein is deleted;
[31] an angiogenesis-suppressing agent (preferably an angiogenesis-suppressing agent in cancer cells) comprising an Int6 protein or an Int6 protein expressing-vector as an active ingredient;
[32] a carrier cell, wherein Int6 gene expression is suppressed and angiogenesis is induced;
[33] the carrier cell of [32], into which an siRNA against an Int6 gene has been introduced;
[34] a therapeutic agent for vascular disease, which comprises the angiogenesis-suppressing agent of [31] or the carrier cell of [32] or [33] as an active ingredient;
[35] a method for producing a therapeutic agent for vascular disease, which comprises the step of introducing an siRNA against an Int6 gene into isolated carrier cells;
[36] a method for treating a vascular disease, which comprises the step of administering an effective amount of any one of:
   (a) an hypoxic stress response-promoting agent of the present invention;
   (b) an angiogenesis-inducing agent of the present invention; and
   (c) a carrier cell of the present invention;
[37] a method for treating vascular disease, which comprises the steps of:
   (a) introducing an siRNA against an Int6 gene to isolated carrier cells; and
   (b) administering an effective amount of the siRNA-introduced carrier cells of step (a); and
[38] an autotransplantation therapy, which comprises the steps of:
   (a) collecting carrier cells;
   (b) introducing an siRNA against an Int6 gene to the collected carrier cells; and
   (c) administering an effective amount of the siRNA-introduced carrier cells of step (a).

### Brief Description of the Drawings

Fig. 1 shows the structures (sequences) of a siRNA expression vector which suppresses the Int6 gene expression.
Fig. 2 shows the suppressive effects of Int6 on the transcriptional activity of HIF2α.
Fig. 3 shows a diagram and photographs indicating the effects of Int6 siRNAs to suppress expression of the endogenous Int6 gene.

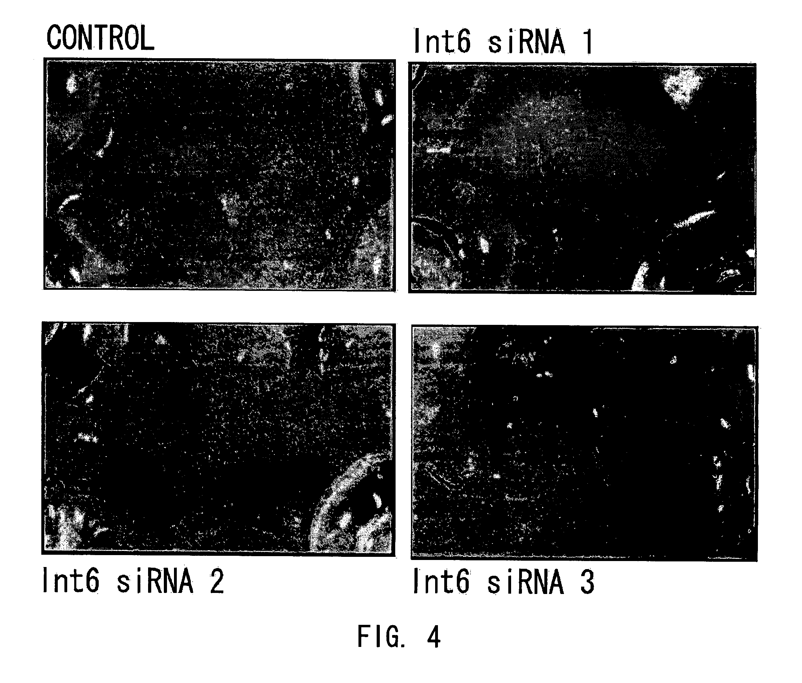

Fig. 4 shows photographs indicating the angiogenesis enhancing effects of the Int6 siRNAs.
Fig. 5 is a graph indicating the results of quantifying the expression of Int6 mRNA in MCF-7 cells cultured for 24 hours after treatment with estradiol (E2), progesterone (Progestin), or the estrogen receptor inhibitor Tamoxifen (4OH-TAM). The codes indicated on the horizontal axis are as follows: A-1, E2(-); A-2, E2 (1 nM); A-4, Progestin (1 nM); A-5, E2 (1 nM) + Progestin (1nM); A-8, 4OH-TAM (1µM); andA-12, E2 (1nM) + 4OH-TAM (1µM).
Fig. 6 shows a group of factors induced by the HIF transcriptional activity, and the ubiquitin-mediated regulatory mechanism.
Fig. 7 summarizes items related to novel HIF2α-binding factors identified by the yeast two-hybrid method.
Fig. 8 shows the relationship between MMTV-induced Int6 mutation and HIF2α.
Fig. 9 shows the basic concept of neovascular bypass therapy by autotransplantation using "siRNA-treated angiogenesis-inducing cells" which secrete a group of angiogenic factors due to introduction of the synthetic Int6-siRNA.
Fig. 10 is a conceptual diagram of neovascular bypass therapy using siRNA-treated angiogenesis-inducing cells.
Fig. 11 shows the binding characteristics of Int6 to HIF2α.
Fig. 12 indicates the HIF2α binding site on Int6.
Fig. 13 shows a graph indicating the transcriptional activity of HIF 1α, HIF2α, and HIF3α for wild-type Int6 and the constitutively inactive (dominant negative) mutant Int6ΔC under normoxic and hypoxic conditions.
Fig. 14 is a photograph showing the results of remarkable induction of subcutaneous angiogenesis in mice by an Int6-siRNA expression vector.
Fig. 15 shows graphs indicating the results of quantitative analyses of angiogenesis by Int6-siRNA. The graph on the left shows the neovascular area (AREA) and the graph on the right shows the length of the new blood vessels (LENGTH).
Fig. 16 shows photographs indicating the histopathological images of blood vessels newly formed due to Int6-siRNA. Arrows in the photographs indicate the newly formed blood vessels.

### Best Mode for Carrying Out the Invention

The present invention showed that substances that suppress (inhibit) Int6 expression or function (activity) have a function of promoting hypoxic stress response by enhancing the functions of HIF2α transcription regulatory factors.

Therefore, the present invention provides hypoxic stress response-promoting agents comprising as an active ingredient, a substance that suppresses Int6 expression or a substance that suppresses Int6 protein function.

The Int6 gene of the present invention is known to be present in various organisms including humans. The Int6 gene is known as the same gene as eIF3e/p48, which is one of the components of a translation regulation factor (translation initiation factor), and genes corresponding to the Int6 gene (homologous genes, homologs) have been identified in humans, mice, rats, frogs, and such.

The Int6 protein of the present invention is preferably a human Int6 protein, but the animal species from which it is derived is not particularly limited, and it may be homologs and such from non-human animal species, for example, mammals including orangutans, chimpanzees, dogs, mice, and Norway rats (rats), birds including chicken, or amphibians including Xenopus. The accession number of the human Int6 gene in the public genetic database GenBank is NM_001568. The names of the genetic databases and the accession numbers in those genetic databases for these non-human animal Int6 homologs are shown below (gb indicates GenBank, emb indicates EMBL, and ref indicates RefSeq database). Orangutan (emb, CR860517.1), dog (ref, XM_532304.1), Norway rat (gb, BC082087.1), mouse (ref, NM_008388.1), chimpanzee (ref, XM_519906.1), chicken (emb, AJ719829.1; ref, NM_001006349.1), and Xenopus (gb, AF162775.1 and AF162775).

The nucleotide sequence of a gene encoding human Int6 is shown as SEQ ID NO: 1. The amino acid sequence of a protein encoded by this nucleotide sequence is shown as SEQ ID NO: 2. Proteins other than those above that are highly homologous to the sequence indicated in the sequence listing of the present application (ordinarily 70% or more, preferably 80% or more, more preferably 90% or more, and most preferably 95% or more) and have a function of Int6 (for example, the function to bind to an HIF2α protein) may be included in the Int6 of the present invention. The protein is, for example, a protein comprising an amino acid sequence with one or more amino acid additions, deletions, substitutions, or insertions in the amino acid sequence of SEQ ID NO: 2, and the number of amino acids that are ordinarily modified is 30 amino acids or less, preferably ten amino acids or less, more preferably five amino acids or less, and most preferably three amino acids or less.

"Int6 protein" of the present invention may be a naturally derived protein, or can be prepared as a recombinant protein using gene-recombination techniques. Naturally derived proteins can be prepared, for example, from an extract solution of cells (tissues) that express an Int6 protein, by methods that use affinity chromatography using antibodies against the Int6 protein. Meanwhile, recombinant proteins can be prepared by culturing cells that have been transformed with an Int6 protein-encoding DNA.

Agents that respond to hypoxic stress in the present invention specifically refer to pharmaceutical agents having a function of increasing the expression of genes that respond to hypoxic stress (in the present description, it may also be referred to as "hypoxic stress-responsive genes"). In more detail, the hypoxic stress-responsive genes in the present invention are genes whose transcription is regulated by HIF2α, and ordinarily, they are genes involved in angiogenesis induction. Usually, multiple hypoxic stress-responsive genes exist in various organisms.

Examples of hypoxic stress-responsive genes of the present invention include genes encoding human vascular endothelial growth factor (VEGF), erythropoietin, insulin growth factor (IGF)-1, glucose transporter carbonic anhydrase IX, and transforming growth factor.

More specifically, substances of the present invention which suppress Int6 expression or function have, for example, a function of enhancing the expression of these genes.

Diseases for which therapeutic effects are expected with the hypoxic stress response-promoting agents of the present invention include diseases accompanying ischemia (typical decrease in blood flow), in which the ischemic condition may be improved by improving blood flow volume through angiogenesis, and include, for example, vascular diseases. More specific examples include ischemic brain diseases such as arteriosclerosis obliterans, restenosis after percutaneous vasodilation treatment, myocardial infarction, cerebral infarction, or intracerebral hemorrhage, neurodegenerative diseases such as Alzheimer's disease, Parkinson's disease, or spinocerebellar degeneration, neurological disorders including traumatic brain injury such as spinal cord injury and brain contusion, liver diseases such as hepatic cirrhosis, and atrophic muscular disorder.

In the present invention, substances that suppress Int6 protein expression include, for example, substances that inhibit the transcription of Int6 or its translation from the transcript. Preferred embodiments of these expression-suppressing substances of the present invention include, for example, compounds (nucleic acids) selected from the group consisting of:
(a) antisense nucleic acids against the Int6 gene transcript or parts thereof;
(b) nucleic acids having a ribozyme activity of specifically cleaving the Int6 gene transcript; and
(c) nucleic acids having an effect of inhibiting the Int6 gene expression by RNAi effects (siRNAs).

"Nucleic acids" in the present invention mean RNAs or DNAs. Moreover, chemically synthesized nucleic acid analogs such as so-called PNAs (peptide nucleic acids) are also included as nucleic acids in the present invention. In PNAs the pentose-phosphate backbone, which is the basic backbone structure of nucleic acids, is substituted with a polyamide backbone, built from glycine units. PNAs have a three-dimensional structure very similar to nucleic acids. Further, so-called LNAs (locked nucleic acids) are also included as nucleic acids in the present invention.

Methods well known to those skilled in the art for inhibiting the expression of a specific endogenous gene include those using antisense technology. Multiple factors contribute to the inhibition of target gene expression by antisense nucleic acids. These factors include, for example, inhibition of transcription initiation through triplex formation; inhibition of transcription through hybrid formation with a sequence at the site of a local open loop structure made by RNA polymerase; inhibition of transcription through hybrid formation with the RNA being synthesized; inhibition of splicing through hybrid formation with a sequence at an intron-exon junction; inhibition of splicing through hybrid formation with a sequence at a site of spliceosome formation; inhibition of transfer from the nucleus to the cytoplasm through hybrid formation with mRNAs; inhibition of splicing through hybrid formation with a sequence at the capping site or poly(A) site; inhibition of translation initiation through hybrid formation with a sequence at the site of binding of the translation initiation factor; inhibition of translation through hybrid formation with a sequence at the ribosome binding site near the initiation codon; inhibition of peptide chain elongation through hybrid formation with a sequence at the site of the translational region or polysome binding site of the mRNAs; and inhibition of gene expression through hybrid formation with a sequence at the site of interaction between proteins and nucleic acids. Thus, antisense nucleic acids inhibit target gene expression by inhibiting various processes, such as transcription, splicing and translation (Hirashima and Inoue, Shin Seikagaku Jikken Koza 2 (New Lecture for Experimental Biochemistry 2), Kakusan IV (Nucleic Acid IV), Replication and Expression of Genes; Ed., Japanese Biochemical Society, Tokyo Kagaku Dozin Co., Ltd., pp. 319-347, 1993).

Antisense nucleic acids used in the present invention may inhibit the expression of an Int6 gene through any one of the actions described above. In one embodiment, an antisense sequence is designed to be complementary to the 5'-untranslated region of an Int6 gene mRNA. Thus such an antisense sequence is expected to effectively inhibit translation of that gene. Sequences complementary to the coding region or 3'-untranslated region can also be used for this purpose. Thus, nucleic acids comprising antisense sequences corresponding to sequences of translated as well as untranslated regions of Int6 genes can be included as antisense nucleic acids used in the present invention. The antisense nucleic acids to be used are ligated downstream of an appropriate promoter, and are preferably ligated with a sequence comprising a transcription termination signal at the 3' end. The nucleic acids thus prepared can be used to transform desired animals by known methods. The antisense nucleic acid sequences are preferably complementary to a sequence of endogenous Int6 gene in the transformed animal or to a portion thereof; however perfect complementarity is not necessary as long as the antisense nucleic acid effectively inhibits gene expression. The transcribed RNAs preferably have 90% or higher complementarity, and most preferably 95% or higher complementarity to the target gene transcript. The length of the antisense nucleic acids used to effectively inhibit target gene (Int6) expression is preferably at least 15 nucleotides or longer and less than 25 nucleotides. However, the antisense nucleotides of the present invention are not limited to this length, and may be 100 nucleotides or longer, or 500 nucleotides or longer, for example.

The antisenses in the present invention are not particularly limited; however, they can be constructed, for example, based on the nucleotide sequence of the Int6 gene obtained from GenBank accession number NM_001568.

Int6 gene expression can also be inhibited using ribozymes or ribozyme-encoding DNAs. Ribozymes refer to RNA molecules with catalytic activity. Ribozymes have a variety of activities, and studies focusing on ribozymes as RNA-cleaving enzymes have allowed the design of ribozymes that cleave RNAs in a site-specific fashion. Ribozymes such as group I intron-type ribozymes and M1 RNA, which are RNase P ribozymes, are 400 nucleotides or more in length. Others such as hammer-head and hairpin ribozymes have active sites comprising about 40 nucleotides (Koizumi, M. and Otsuka, E., Tanpakushitsu Kakusan Koso (Protein, Nucleic acid, and Enzyme), 35:2191, 1990).

For example, the autolytic domains of hammer-head ribozymes cleave the 3' side of C15 in the sequence G13U14C15. Base pairing between U14 and A9 plays an important role in this activity, and A15 or U15 can be cleaved instead of C15 (Koizumi, M. et al., FEBS Lett, 228:228, 1988). Restriction enzyme-like RNA-cleaving ribozymes that recognize the target RNA sequences UC, UU or UA can be produced by designing the ribozymes such that a substrate binding site complements the RNA sequence near the target site (Koizumi, M. et al., FEBS Lett, 239:285, 1988; Koizumi, M. and Otsuka, E., Tanpakushitsu Kakusan Koso (Protein, Nucleic acid, and Enzyme), 35:2191, 1990; and Koizumi, M. et al., Nucl. Acids Res., 17:7059, 1989).

Hairpin ribozymes can also be used for the purposes of the present invention. Such ribozymes are found, for example, in the minus strand of tobacco ring spot virus satellite RNA (Buzayan, J. M., Nature, 323:349, 1986). Target specific RNA-cleaving ribozymes can also be produced from hairpin ribozymes (Kikuchi, Y and Sasaki, N., Nucl. Acids Res., 19:6751, 1991; Kikuchi, H., Kagaku to Seibutsu (Chemistry and Biology), 30:112, 1992). Thus, in the present invention Int6 gene expression can be inhibited by specifically digesting the Int6 gene transcript using a ribozyme.

Methods using ribozymes have been used as methods for specifically suppressing gene expression (Beger C. et al., Proc. Natl. Acad. Sci. USA 98:130-135, 2001),and recently RNA interference (hereinafter abbreviated as "RNAi") is attracting attention as a method that can more effectively suppress gene expression. Nucleic acids with inhibitory activity due to RNAi effect are also generally called siRNAs. RNAi is a phenomenon in which destruction of a target gene mRNA can be induced and target gene expression can be suppressed by introducing cells and the like with short double stranded RNAs (hereinafter abbreviated as "dsRNAs") consisting of sense RNAs homologous to an mRNA of the target gene and antisense RNAs consisting of their complementary sequences. Thus, RNAi can suppress the expression of target genes, and is therefore drawing attention as a simple method for knocking out genes, replacing conventional gene destruction methods using complicated and inefficient homologous recombination, and as a method applicable to gene therapy. RNAs used for RNAi do not necessarily have to be completely identical to an Int6 gene or to a partial region of the gene; however, complete homology is preferred.

Preferable embodiments of the nucleic acids of (c) mentioned above (siRNAs), are double stranded RNAs with RNAi effect (siRNAs) on an Int6 gene. More specific examples comprise double stranded RNAs (siRNAs) comprising sense and antisense RNAs that correspond to a partial sequence of a nucleotide sequence of SEQ ID NO: 1.

Although details of the mechanism of RNAi are still partially unclear, it is believed that an enzyme called DICER (a member of the RNase III nuclease family) contacts the double stranded RNAs, which are degraded into small fragments called small interfering RNAs, or siRNAs. The double stranded RNAs with RNAi effect of the present invention also comprise double stranded RNAs prior to such degradation by DICER. In other words, because even a long RNA, which by itself has no RNAi effect, is expected to be degraded within cells to form siRNAs that have an RNAi effect, the length of the double stranded RNAs of the present invention is not particularly limited.

For example, a long double stranded RNA corresponding to a full length, or almost full length region of an mRNA of an Int6 gene of the present invention may be pre-degraded with DICER, and those degradation products can be used as agents of the present invention. Such degradation products are expected to comprise double stranded RNA molecules with RNAi effect (siRNAs). In such methods, it is not particularly necessary to select mRNA regions expected to have an RNAi effect. Thus, it is not particularly necessary to precisely define the region(s) on an mRNA of an Int6 gene of the present invention that have an RNAi effect.

With respect to the above RNA molecules, molecules that have a closed structure on one end, for example, siRNAs comprising a hairpin structure (shRNAs), are also comprised in the present invention. Thus, single stranded RNA molecules that can form a double stranded RNA structure within the molecule are also comprised in the present invention.

The "double stranded RNA that can be suppressed by RNAi effects" of the present invention can be prepared appropriately by those skilled in the art based on the nucleotide sequence of the Int6 gene of the present invention which becomes the target of the double stranded RNA. For example, a double stranded RNA of the present invention can be prepared based on the nucleotide sequence of SEQ ID NO: 1. More specifically, those skilled in the art can suitably select any continuous RNA region of an mRNA, which is a transcript of the sequence based on the nucleotide sequence of SEQ ID NO: 1, and prepare a double stranded RNA corresponding to this region within the range of ordinarily performed experiments. Furthermore, those skilled in the art can also use known methods to appropriately select siRNA sequences having stronger RNAi effects from mRNA sequences which are transcripts of this sequence. If one of the strands (for example, the nucleotide sequence of SEQ ID NO: 1) is identified, those skilled in the art can easily find out the nucleotide sequence of the other strand (complementary strand). siRNAs can be prepared appropriately by those skilled in the art using a commercially available nucleic acid synthesizer. For synthesis of desired RNAs, a general contract synthesis service can be used.

Furthermore, DNAs (vectors) that can express the RNAs of the present invention are also included in the preferred embodiments of compounds that can suppress the expression of the Int6 gene of the present invention. For example, DNAs (vectors) that can express the double stranded RNAs of the present invention are DNAs that comprise a structure in which a DNA encoding one strand of the double stranded RNA and a DNA encoding the other strand of the double stranded RNA are linked with a promoter so that each of them can be expressed. Such DNAs of the present invention can be prepared appropriately by those skilled in the art using general genetic engineering techniques. More specifically, expression vectors of the present invention can be prepared by appropriately inserting DNAs encoding the RNAs of the present invention into various known expression vectors.

The expression-suppressing substances of the present invention include, for example, compounds that suppress Int6 expression by binding to an expression regulatory region on Int6 (for example, a promoter region). Such compounds can be obtained, for example, by a screening method that uses the Int6 promoter DNA fragment, and uses the activity to bind the DNA fragment as an indicator. Furthermore, whether a compound of interest can suppress the expression of Int6 of the present invention can be suitably determined by those skilled in the art using well known methods, for example, reporter assay methods.

Furthermore, DNAs (vectors) that can express the RNAs of the present invention are also included in the preferred embodiments of the compounds that can suppress the expression of Int6 gene of the present invention. For example, DNAs (vectors) that can express the double stranded RNAs of the present invention are DNAs that comprise a structure in which a DNA encoding one strand of the double stranded RNA and a DNA encoding the other strand of the double stranded RNA are linked with a promoter so that each of them can be expressed. Such DNAs of the present invention can be prepared appropriately by those skilled in the art using general genetic engineering techniques. More specifically, expression vectors of the present invention can be prepared by appropriately inserting DNAs encoding the RNAs of the present invention into various known expression vectors.

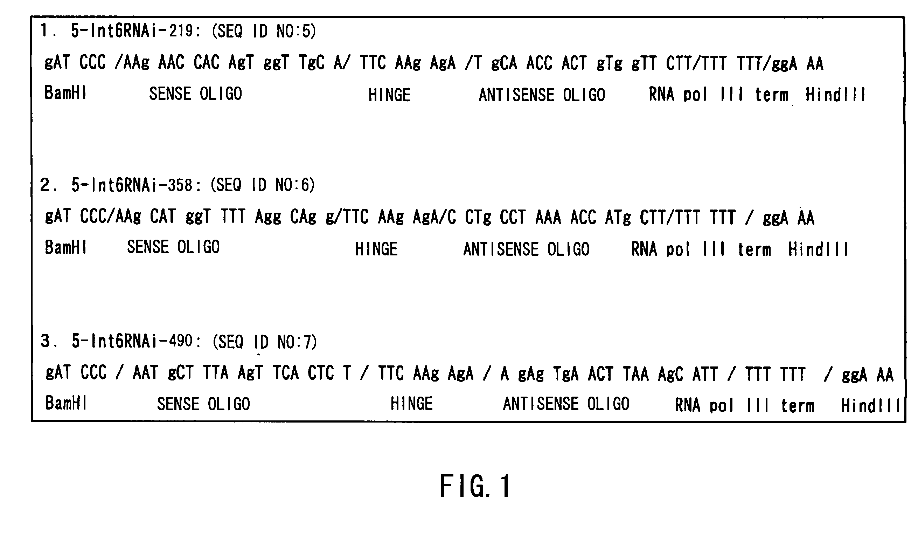

A preferred embodiment of the vector of the present invention includes vectors expressing siRNAs that suppress Int6 expression. For example, nucleotide sequences of regions that express siRNAs in these vectors may include the sequences (SEQ ID NOs: 5 to 7) shown in Fig. 1, but are not particularly limited thereto.

A preferred example of the siRNAs of the present invention is an siRNA comprising a structure in which the respective sequences of the regions described as sense oligo and antisense oligo in each of the nucleotide sequences shown in Fig. 1 are hybridized.

The expression-suppressing substances of the present invention include, for example, compounds that suppress the Int6 expression by binding to an expression regulatory region of Int6 (for example, a promoter region). Such compounds can be obtained, for example, by a screening method that uses the activity to bind a DNA fragment of the Int6 promoter as an indicator. Furthermore, whether a compound of interest can suppress the expression of Int6 of the present invention can be suitably determined by those skilled in the art using well known methods, for example, reporter assay methods.

The present invention also provides hypoxic stress response-promoting agents, comprising a substance that suppresses Int6 protein function as an active ingredient. Since the Int6 protein binds to HIF2α and has a function of suppressing HIF2α function, suppressing Int6 protein function activates HIF2α function and enhances the function of transcribing hypoxic stress-responsive genes. Therefore, substances that suppress Int6 protein function are considered useful as hypoxic stress response-promoting agents.

Substances of the present invention that suppress Int6 protein function include, for example, the following compounds:
(a) antibodies that bind to an Int6 protein;
(b) low-molecular-weight compounds that bind to an Int6 protein; and
(c) compounds that inhibit the binding activity between Int6 protein and HIF2α protein.

The antibodies that bind to Int6 proteins (anti- Int6 antibodies) can be prepared by methods known to those skilled in the art. When the antibodies are polyclonal antibodies, they can be prepared, for example, using the following methods: Antiserum is obtained by immunizing small animals such as rabbits with a natural Int6 protein, or a (recombinant) Int6 protein expressed as a fusion protein with GST in microorganisms such as *Escherichia coli,* or a partial peptide thereof. Antibodies are purified from the serum using, for example, ammonium sulfate precipitation, protein A columns, protein G columns, DEAE ion-exchange chromatography, affinity columns on to which an Int6 protein or synthetic peptide thereof is immobilized, etc. Alternatively, monoclonal antibodies can be produced by, for example, immunizing small animals such as mice with an Int6 protein or partial peptide thereof, removing the spleen, gently grinding the spleen to separate cells, fusing the cells with mouse myeloma cells using a reagent such as polyethylene glycol, and then screening the fused cells (hybridomas) to select clones that produce antibodies that bind to the Int6 protein. These hybridomas are then transplanted into a mouse peritoneal cavity and ascites are collected from the same mouse. The monoclonal antibodies thus prepared can be purified using, for example, ammonium sulfate precipitation, protein A columns, protein G columns, DEAE ion-exchange chromatography, affinity columns on to which an Int6 protein or synthetic peptide is immobilized, etc.

The antibodies of the present invention are not particularly limited so long as they bind to an Int6 protein of the present invention, and include, in addition to the polyclonal antibodies and monoclonal antibodies, human antibodies, humanized antibodies prepared by genetic recombination, and their antibody fragments and modified antibodies.

Int6 proteins of the present invention that are to be used as sensitizing antigens for obtaining antibodies are not limited in terms of the animal species from which they are derived, but proteins derived from mammals such as mice or humans are preferred, and human-derived proteins are particularly preferred. Human derived proteins can be appropriately obtained by those skilled in the art using genetic sequences or amino acid sequences disclosed in the present description.

Proteins to be used as an immunization antigens in the present invention may be full-length proteins or partial peptides derived from those proteins. Such partial protein peptides include, for example, protein amino (N)-terminal fragments and carboxyl (C)-terminal fragments. As used herein, "antibodies" refer to antibodies which react with full-length proteins or fragments thereof.

In addition to obtaining the above hybridomas by immunizing non-human animals with antigens, hybridomas producing desired human antibodies with protein binding activity can also be prepared *in vitro* by sensitizing human lymphocytes, for example, human lymphocytes infected with EB virus, with a protein, cells expressing a protein, or a lysate of those cells, and fusing these sensitized lymphocytes with immortalized human myeloma cells, for example, U266 cells.

Anti-Int6 protein antibodies of the present invention inhibit Int6 protein function by binding to Int6 protein, and are expected to have, for example, effects of improving or treating vascular diseases. When the obtained antibodies are used for the purpose of administering to humans (antibody therapy), human antibodies or humanized antibodies are preferred because of their lower immunogenicity.

Furthermore, the present invention includes low-molecular-weight substances (low-molecular-weight compounds) that bind to an Int6 protein as substances that can inhibit Int6 protein function. The low-molecular-weight substances of the present invention that bind to an Int6 protein may be naturally-derived or artificial compounds. Ordinarily, they are compounds that can be produced or obtained by using methods well known to those skilled in the art. Furthermore, the compounds of the present invention can be obtained by the screening methods to be described.

Examples of the above-mentioned low-molecular-weight compounds of (b) that bind to Int6 protein and suppress its function include compounds that have high affinity for Int6.

A preferred embodiment of the substances of the present invention that suppress Int6 function include, for example, compounds that inhibit the binding (interaction) between HIF2α and Int6. These compounds increase the amount of free HIF2α functioning as a transcription factor by inhibiting the binding between HIF2α and Int6, and as a result, they are considered to promote the transcription of hypoxic stress-responsive genes.

Examples of substances of the present invention that can suppress Int6 protein function also include Int6 protein mutants (Int6 dominant negative proteins) that have a dominant-negative property for Int6 protein. "Int6 protein mutants that have a dominant-negative property for Int6 protein" refers to proteins that have the function of eliminating or decreasing the activity of the endogenous wild-type protein by expressing genes encoding the proteins. More specifically, examples of the Int6 dominant negative proteins include proteins that have lost their activity to bind HIF2α.

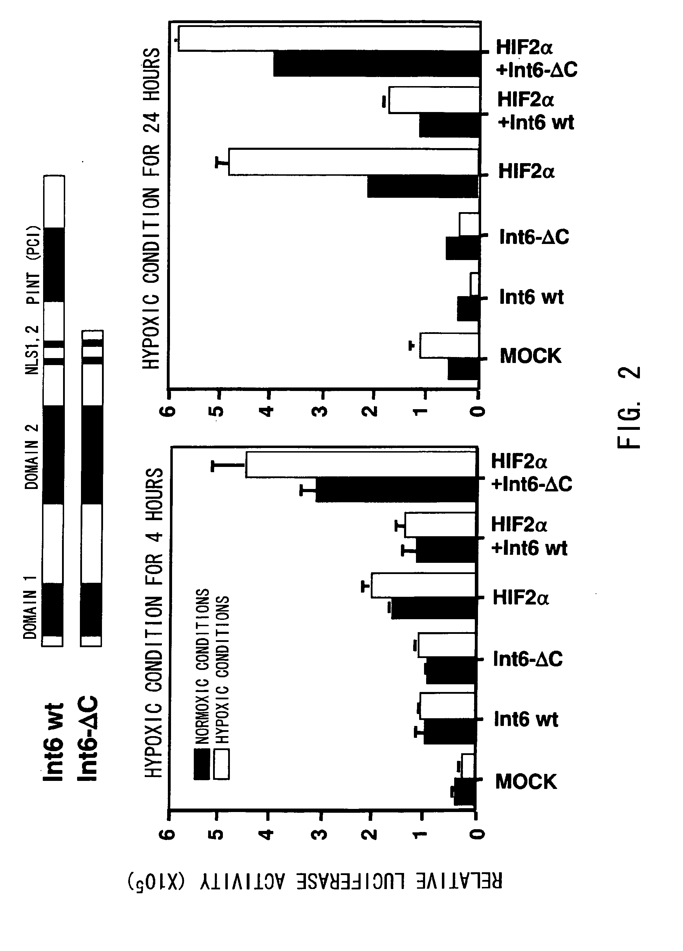

An example of the Int6 dominant negative proteins include a protein in which the C terminus of Int6 protein has been deleted (Int6-ΔC). Specifically, this Int6-ΔC is, for example, an Int6 protein mutant in which the PINT region in the C-terminal region of Int6 protein has been deleted (see Fig. 2).

Hypoxic stress response-promoting agents provided by the present invention are expected to have therapeutic effects for vascular diseases since they have effects of inducing (promoting) angiogenesis.

Therefore, the present invention provides therapeutic agents (pharmaceutical compositions) for vascular diseases, which comprise a hypoxic stress response-promoting agent of the present invention as an active ingredient.

Substances of the present invention that enhance Int6 expression or its function are expected to have the function of decreasing hypoxic stress response by decreasing the function of the HIF2α transcription regulatory factors.

Therefore, the present invention provides hypoxic stress response-suppressing agents, which comprise as an active ingredient a substance that enhances Int6 protein expression or its function. "Enhancement of protein expression" includes enhancement of gene transcription, and enhancement of translation from transcript.

Examples of such substances include substances that enhance the binding (interaction) between Int6 and HIF2α. These substances can be obtained appropriately by screening for substances that enhance the binding activity using the binding activity between Int6 protein and HIF2α protein as an indicator.

Since the hypoxic stress response-suppressing agents provided by the present invention have effects of suppressing (inhibiting) angiogenesis, they are expected to have therapeutic effects for diseases related to angiogenesis (angiogenesis-related diseases) such as cancers. Examples of cancers for which therapeutic effects are expected preferably include breast cancer, colon cancer, intraoral cancer, pharyngeal and laryngeal cancer, esophageal cancer, gastric cancer, liver cancer, renal cancer, bladder cancer, and uterine cancer. In addition to cancers, diseases for which therapeutic effects can be expected from suppression of angiogenesis include, for example, diseases in which abnormal blood vessels increase in retina such as diabetic retinosis, and pulmonary hypertension. Suppression of the function of the HIF2α transcription regulatory factor not only suppresses (inhibits) angiogenesis, but may also have effects on polycythemia or abnormal glucose metabolism in diabetes.

The present inventors have discovered that female hormones suppress Int6 expression. More specifically, female hormones enhance the activity of HIF2α to promote the transcription of hypoxic stress-responsive genes by suppressing Int6 gene expression, and as a result they are considered to promote angiogenesis. Therefore, female hormones are effective for treating, for example, diseases whose pathology may be improved by improving blood flow volume (for example, arteriosclerosis obliterans).

Female hormones are preferred substances for suppressing Int6 expression in the present invention. More specifically, examples of female hormones are estrogen, progesterone, and such. Meanwhile, in addition to female hormones, substances that enhance Int6 protein expression or its function include female hormone secretion-promoting agents, agonists of female hormone receptors and such.

Female hormone-suppressing agents are expected to decrease angiogenesis by enhancing Int6 gene expression and thereby decreasing the activity of HIF2α to promote the transcription of hypoxic stress-responsive genes. Therefore, female hormone-suppressing agents are useful for treating, for example, diseases whose pathology may be improved by suppressing angiogenesis (for example, cancer diseases).

The present invention also relates to carrier cells that have suppressed Int6 gene expression and induced angiogenesis. Such cells are expected to show therapeutic effects for vascular diseases. Examples of "carrier cells" of the present invention include fibroblasts and glial cells. Int6 gene expression can be suppressed, for example, using siRNAs against Int6 gene.

The present invention provides therapeutic agents for angiogenesis-related diseases (for example, anticancer agents and antitumor pharmaceutical compositions) which comprise a hypoxic stress response-suppressing agent of the present invention as an active ingredient. Examples of a preferred embodiment of the therapeutic agents for angiogenesis-related diseases include therapeutic agents for angiogenesis-related diseases comprising a female hormone-suppressing agent as an active ingredient.

Furthermore, the present invention provides non-human transgenic animals (in the present description, it may be described as "non-human transgenic animal(s)", "knockout non-human animal(s)", or simply "animal(s)") whose Int6 gene expression is artificially suppressed.

The non-human transgenic animals of the present invention can be used favorably, for example, to screen for pharmaceutical agents to treat diseases that require regulation of hypoxic stress response. They are also very useful as experimental model animals for research on elucidating the mechanism of these diseases and hypoxic stress response.

The non-human transgenic animals of the present invention include so-called "knockdown animals" whose gene expression is suppressed by the action of antisense RNA or siRNA.

Examples of the conditions in which "Int6 gene expression is artificially suppressed" in the present invention include (1) conditions in which expression of Int6 gene is suppressed by the presence of genetic modifications such as nucleotide insertion, deletion, or substitution in one or both of the gene pair, and (2) conditions in which gene expression is suppressed by the action of the nucleic acids of the present invention (for example, antisense RNA or siRNA).

"Suppressed" in the present invention includes cases in which Int6 gene expression is completely suppressed, and cases in which the level of Int6 gene expression in the animals of the present invention is significantly decreased compared to the Int6 gene expression level in a wild-type animal.

The condition (1) mentioned above also includes cases in which the expression of only one of the genes in the pair of Int6 genes is suppressed. Regions where gene modifications are present are not particularly limited in the present invention so long as they are regions that suppress gene expression, and examples include exon regions and promoter regions.

The non-human transgenic animals of the present invention can be prepared by those skilled in the art using generally known genetic engineering techniques. For example, gene knockout mice can be prepared as follows. First, isolate a DNA comprising an exon region of Int6 gene of the present invention from mice, and insert a suitable marker gene into this DNA fragment to construct a targeting vector. Introduce this targeting vector into a mouse ES cell line by the electroporation method or the like, and select cell lines that have undergone homologous recombination. Marker genes to be inserted are preferably antibiotic resistance genes such as the neomycin resistance gene. When an antibiotic resistance gene is inserted, the cell line that has undergone homologous recombination can be selected just by culturing in a medium containing the antibiotic. Alternatively, the thymidine kinase gene and such can be linked to a targeting vector for more efficient selection. This eliminates cell lines that have undergone non-homologous recombination. It is also possible to efficiently obtain cell lines in which one member in the gene pair of a gene of the present invention has been inactivated, by assaying homologous recombinants by PCR and Southern blotting.

When selecting cell lines in which homologous recombination has taken place, since there is a risk of unknown gene destruction in sites other than the site of homologous recombination due to gene insertion, it is preferable to use multiple clones to generate chimera. Chimeric mice can be obtained by injecting the obtained ES cell lines into mouse blastoderms. By crossing these chimeric mice, mice in which one of a gene pair of an Int6 gene of the present invention is inactivated can be obtained. Further, by crossing these mice, mice in which both of the gene pair of a gene of the present invention are inactivated can be obtained. Similar procedures may be employed to genetically modify animals other than mice for which ES cells are established.

The knockout animals of the present invention are preferably knockout (knockdown) animals in which Int6 gene expression is suppressed by introducing the nucleic acids of the present invention into the non-human animals.

The above knockdown animals can be constructed by introducing non-human animals with a vector structured such that the nucleic acids (antisense RNAs, siRNAs, or such) of the present invention can be expressed.

For example, non-human animals with decreased Int6 expression can be prepared by cervicodorsal subcutaneous injection of vectors that express siRNAs comprising the nucleotide sequence of SEQ ID NO: 5, 6 or 7 into non-human animals. More specifically, non-human transgenic animals of the present invention can be prepared by methods described in the Examples to be described later.

The type of transgenic animals of the present invention is not particularly limited so long as they are non-human animals, but they are usually mammals and preferably primates. More specifically, animals of the present invention are preferably mice, rats, flies, nematodes (*C. Elegans),* cattle, pigs, birds, or sheep, and more preferably mice. When a gene corresponding to Int6 is present in plants, it is also possible to prepare knockdown (transgenic) plants targeting this gene.

In a preferred embodiment, the non-human transgenic animals of the present invention are, without being limited thereto, animals in which Int6 gene expression is suppressed by the action of any of the nucleic acids of:
(a) antisense nucleic acids against an Int6 gene transcript or a part thereof;
(b) nucleic acids having a ribozyme activity of specifically cleaving an Int6 gene transcript; and
(c) nucleic acids having a function of inhibiting Int6 gene expression by RNAi effect.

Since the expression of Int6 gene is suppressed and angiogenesis is induced in non-human transgenic animals of the present invention, the animals are very useful, for example, in screening for substances or pharmaceutical agents that inhibit angiogenesis (therapeutic agents for angiogenesis-related diseases (for example, anticancer agents)) and analyzing the mechanism of angiogenesis.

Furthermore, the present invention provides methods that use the level of Int6 expression or activity as an indicator in screening for therapeutic agents of vascular diseases.

Compounds that decrease (suppress) the expression level of Int6 gene are anticipated to be potential pharmaceutical agents for treating vascular diseases. On the contrary, compounds that increase (enhance) the level of Int6 gene expression are anticipated to be potential pharmaceutical agents for treating diseases caused by angiogenesis, for example, cancers.

Preferred embodiments of the methods of the present invention are methods of screening for therapeutic agents for vascular diseases, which comprise the steps of:
(a) contacting a test compound with Int6 gene-expressing cells;
(b) measuring the level of Int6 gene expression in the cells; and
(c) selecting a compound that decreases the expression level compared with that measured in the absence of the test compound.

Other embodiments of the methods of the present invention are methods of screening for therapeutic agents for angiogenesis-related diseases (for example, anticancer agents), which comprise the steps of:
(a) contacting a test compound with Int6 gene-expressing cells;
(b) measuring the level of Int6 gene expression in the cells; and
(c) selecting a compound that increases the expression level compared with that measured in the absence of the test compound.

In the above-mentioned methods, first, a test compound is contacted with cells expressing an Int6 gene. "Cells" that are used may be cells derived from humans, mice, rats, and such but are not particularly limited to cells derived from them, and cells of microorganisms such as *Escherichia coli* and yeast that have been transformed to express Int6 can also be used. Cells expressing an endogenous Int6 gene or cells that express the gene by introduction of a foreign Int6 gene can be used as the "cells expressing an Int6 gene". Normally, cells expressing a foreign Int6 gene can be prepared by introducing into host cells an expression vector inserted with an Int6 gene. These expression vectors can be prepared by conventional genetic engineering techniques.

The test compounds used in the present methods are not particularly limited and include, for example, single compounds such as natural compounds, organic compounds, inorganic compounds, proteins, and peptides, as well as chemical libraries, the expressed products from gene libraries, cell extracts, supernatants from cell cultures, bacterial fermentation products, marine organism extracts, and plant extracts.

Typically, but without limitation, a test compound is contacted with cells expressing an Int6 gene by adding the test compound to a culture medium of the cells expressing the Int6 gene. When the test compound is a protein, the contact can be achieved by introducing into the cells a DNA vector that allows protein expression.

The next step of these methods comprises determining the expression level of the Int6 gene. Herein, the phrase "gene expression" refers to both transcription and translation. The gene expression level can be determined using methods known to those skilled in the art. For example, mRNAs can be extracted from cells expressing an Int6 gene according to conventional methods, and by using this mRNA as a template, the transcriptional level of the gene can be determined using Northern hybridization or RT-PCR. Alternatively, the translational level of the gene can be determined by collecting protein fractions from the cells expressing the Int6 gene, and then expression of the Int6 protein can be detected using an electrophoresis method such as sodium dodecyl sulfate-polyacrylamide gel electrophoresis (SDS-PAGE). Furthermore, the translational level of the gene can be determined by detecting the expression of the Int6 protein by Western blotting analysis using antibodies against the protein. The types of antibodies used for Int6 protein detection are not limited, as long as the protein can be detected. Such antibodies include, for example, both monoclonal and polyclonal antibodies.

Next in the present methods, compounds that increase or decrease the expression level compared with that measured when the test compounds are not contacted (controls) are selected. Compounds that decrease the expression level become therapeutic agents for vascular diseases, while compounds that increase the expression level become therapeutic agents against diseases for which therapeutic effects may be exhibited by suppressing angiogenesis (for example, cancers).

Another embodiment of the screening methods of the present invention are methods for selecting compounds which decrease or increase the expression level of an Int6 gene of the present invention by using the expression of a reporter gene as an indicator.

Preferred embodiments of the above methods of the present invention are methods of screening for therapeutic agents for vascular diseases comprising the following steps (a) to (c);
(a) contacting a test compound with cells or an extract solution of cells comprising a DNA which comprises a structure in which a reporter gene is operably linked to the transcriptional regulatory region of an Int6 gene;
(b) measuring the expression level of the reporter gene; and
(c) selecting a compound that decreases the expression level compared with that measured in the absence of the test compound.

Furthermore, other embodiments of the above methods of the present invention are methods of screening for therapeutic agents for angiogenesis-related diseases (for example, anticancer agents) comprising the following steps (a) to (c):
(a) contacting a test compound with cells or extract solution of cells comprising a DNA which comprises a structure in which a reporter gene is operably linked to a transcriptional regulatory region of an Int6 gene;
(b) measuring the expression level of the reporter gene; and
(c) selecting a compound that increases the expression level compared with that measured in the absence of the test compounds.

In these methods, test compounds are first contacted with cells (or extracts of these cells) that comprise a DNA with a structure in which a reporter gene is operably linked to a transcriptional regulatory region of an Int6 gene. As used herein, the phrase "operably linked" means that the transcriptional regulatory region of an Int6 gene is linked to a reporter gene in such a way as to induce reporter gene expression when a transcriptional factor binds to the transcriptional regulatory region of the Int6 gene. Thus, even when the reporter gene is connected with another gene and thus forms a fusion protein with that gene product, such a connection can be expressed by the phrase "operably linked", as long as the expression of the fusion protein is induced when the transcriptional factor binds to the transcriptional regulatory region of the Int6 gene. Using known methods and based on cDNA nucleotide sequences of Int6 genes, those skilled in the art can obtain from the genome the transcriptional regulatory region of Int6 genes.

The types of reporter genes used in these methods are not limited, as long as its expression can be detected. Such reporter genes include, for example, the CAT gene, lacZ gene, luciferase gene, and GFP gene. The "cells that comprise a DNA structured such that a reporter gene is operably linked to a transcriptional regulatory region of an Int6 gene" include, for example, cells introduced with a vector inserted with such a structure. Those skilled in the art can prepare such vectors using routine genetic engineering techniques. Such a vector can be introduced into cells using conventional methods, for example, using calcium phosphate precipitation, electroporation, the lipofectamine method, microinjection, and so on. The "cells that comprise a DNA structured such that a reporter gene is operably linked to a transcriptional regulatory region of an Int6 gene" also include cells with that structure has been inserted into their chromosome. The DNA structures can be inserted into chromosomes using methods routinely used by those skilled in the art, for example, gene transfer methods using homologous recombination.

"Extracts of cells that comprise a DNA structured such that a reporter gene is operably linked to a transcriptional regulatory region of an Int6 gene" include, for example, mixtures prepared by adding DNAs to cell extracts included in commercially available *in vitro* transcription translation kits, wherein the added DNAs comprise structures such that a reporter gene is operably linked to a transcriptional regulatory region of an Int6 gene.

In this method, "contact" can be achieved by adding test compounds to the culture media of "cells that comprise a DNA structured such that a reporter gene is operably linked to a transcriptional regulatory region of an Int6 gene", or by adding test compounds to the above commercially available cell extracts, which comprise such DNAs. When the test compounds are proteins, the contact can also be achieved, for example, by introducing the cells with a DNA vector that expresses those proteins.

The next step in these methods is determining the level of reporter gene expression. The expression level of a reporter gene can be determined by methods that depend on the type of reporter gene, which are known to those skilled in the art. For example, when the reporter gene is the CAT gene, its expression level can be determined by detecting the acetylation of chloramphenicol, which is mediated by the CAT gene product. When the reporter gene is the lacZ gene, expression level can be determined by detecting color development in a chromogenic compound, mediated by the catalytic action of the lacZ gene expression product. When the reporter gene is a luciferase gene, the expression level can be determined by detecting the fluorescence of a fluorescent compound, mediated by the catalytic action of the luciferase gene expression product. Alternatively, when the reporter gene is the GFP gene, the expression level can be determined by detecting the fluorescence of the GFP protein.

Next, in the present methods, compounds that decrease (suppress) or increase (enhance) the measured expression level of the reporter gene compared with that measured in the absence of the test compound are selected. Compounds that decrease (suppress) the expression level become pharmaceutical agents for treating vascular diseases, while compounds that increase (enhance) the expression level become therapeutic agents for diseases on which suppression of angiogenesis may exhibit therapeutic effects (for example, cancers).

Other embodiments of the screening methods of the present invention are methods that use the Int6 protein activity as an indicator. The methods are, for example, methods of screening for therapeutic agents for vascular diseases, which comprise the steps of:
(a) contacting a test compound with an Int6 protein, or cells or an extract solution of cells expressing the protein;
(b) measuring the activity of the protein; and
(c) selecting a compound that decreases the activity of the protein compared with that measured in the absence of the test compound.

Other embodiments of the above-mentioned methods of the present invention are, for example, methods of screening for therapeutic agents for angiogenesis-related diseases (for example, anticancer agents), which comprise the steps of:
(a) contacting a test compound with an Int6 protein, or cells or an extract solution of cells expressing the protein;
(b) measuring the activity of the protein; and
(c) selecting a compound that increases the activity of the protein compared with that measured in the absence of the test compound.

In the present method, first, test compounds are contacted with an Int6 protein, or cells or an extract solution of cells expressing the protein.

Next, activity of the Int6 protein is measured. Examples of the activity of Int6 protein include binding activity (interaction activity) with HIF2α. This activity can be appropriately measured by those skilled in the art using known methods such as the immunoprecipitation method or yeast two-hybrid method.

The region of amino acids in HIF2α that is involved in the binding with Int6 is the amino acid region (SEQ ID NO: 4) of positions 571 to 700 of HIF2α. Therefore, for example, the binding activity can be measured by using polypeptide fragments comprising the above amino acid region of HIF2α protein. The nucleotide sequence of HIF2α gene is shown as SEQ ID NO: 3, and the amino acid sequence of the protein encoded by this gene is shown as SEQ ID NO: 4. The accession number of HIF2α gene in the public gene database is NM_001430.

Furthermore, compounds that decrease (suppress) or increase (enhance) the activity of this protein compared with that measured in the absence of the test compound are selected. Compounds that decrease (suppress) the activity become pharmaceutical agents for treating vascular diseases, while compounds that increase (enhance) the activity become therapeutic agents for diseases on which suppression of angiogenesis may exhibit therapeutic effects (for example, cancers).

Examples of other embodiments of the screening methods of the present invention include methods that use binding activity between Int6 protein and HIF2α protein as an indicator. The Int6 protein of the present invention is a protein that has the activity to bind (interact with) a HIF2α protein. Therefore, it is possible to screen for pharmaceutical agents for treatment of vascular diseases or therapeutic agents for diseases on which suppression of angiogenesis may exhibit therapeutic effects (for example, cancers), by selecting substances that enhance this binding activity or substances that decrease (suppress) this binding activity, using the binding activity between Int6 protein and HIF2α protein as an indicator.

The method of the present invention is, for example, a method of screening for therapeutic agents for vascular diseases, which comprises the steps of:
(a) contacting a test compound with an Int6 protein and an HIF2α protein;
(b) measuring the binding activity between the Int6 protein and the HIF2α protein; and
(c) selecting a compound that decreases the binding activity compared with that measured in the absence of the test compound.

The above-mentioned method of the present invention includes, for example, a method of screening for therapeutic agents for angiogenesis-related diseases (for example, anticancer agents), which comprises the steps of:
(a) contacting a test compound with an Int6 protein and an HIF2α protein;
(b) measuring the binding activity between the Int6 protein and the HIF2α protein; and
(c) selecting a compound that increases the binding activity compared with that measured in the absence of the test compound.

In the present method, first, a test compound is contacted with an Int6 protein and an HIF2α protein. Next, the binding activity between the Int6 protein and HIF2α protein is measured.

Usually, the binding (interaction) activity between the Int6 protein and HIF2α protein can be simply measured by those skilled in the art, and the binding activity in step (b) above can be measured appropriately using conventional methods such as coimmunoprecipitation method.

Int6 protein and HIF2α protein used in the method are preferably wild-type proteins that do not contain mutations, but as long as they have interaction activity, they may be proteins (polypeptides) that have parts of their amino acid sequences substituted or deleted.

Int6 protein used in the above-mentioned method is preferably a wild-type protein (full-length protein) that does not contain mutations, but so long as it maintains binding activity with HIF2α protein, it may be a partial peptide fragment, or a protein (polypeptide) that has parts of its amino acid sequence substituted or deleted.

The region of Int6 protein involved in the binding with HIF2α protein is usually a region called "Domain 1" which exists at the N terminus. Therefore, Int6 protein used in the above-mentioned method may be a partial peptide fragment of the protein comprising "Domain 1". "Domain 1" is a region corresponding to positions 1 to 80 in the amino acid sequence of Int6 protein shown as SEQ ID NO: 2.

Next, the above-mentioned method selects compounds that decrease (suppress) or increase (enhance) the binding activity compared with that measured in the absence of the test compound. Compounds that decrease (suppress) the binding activity become pharmaceutical agents for treating vascular diseases, while compounds that increase (enhance) the binding activity become therapeutic agents for diseases on which suppression of angiogenesis may exhibit therapeutic effects (for example, cancers).

In the above-mentioned non-human transgenic animals of the present invention, Int6 gene expression is suppressed, and as a result, significant angiogenic effects are observed. Compounds that suppress angiogenesis can be screened efficiently by using these non-human transgenic animals. Compounds obtained by this screening method are anticipated to be potential therapeutic agents for angiogenesis-related diseases (for example, anticancer agents).

The screening methods of the present invention are methods that use non-human transgenic animals of the present invention, and as an indicator, use angiogenic effects or the expression or function (activity) of Int6.

The above-mentioned methods are, for example, methods of screening for therapeutic agents for vascular diseases, which comprise the steps of:
(a) administering a test compound to a non-human transgenic animal of the present invention;
(b) measuring the level of Int6 expression or activity in the non-human transgenic animal, or the angiogenic effect in the animal; and
(c) selecting a compound that decreases the angiogenic effect, or a compound that increases the level of Int6 expression or activity compared with that when the test compound is not administered (control).

In the present method, first, a test compound is administered to non-human transgenic animals. The test compound can be administered by oral or parenteral administration, but when the test compound is a peptide, parenteral administration is preferred. More specifically, the administration may include vascular administration, transnasal administration, transpulmonary administration, and transdermal administration. Examples of vascular administration include intravenous injection, intramuscular injection, intraperitoneal injection, and subcutaneous injection, and the administration may be systemic or local.

When DNAs are the test compounds, they can be administered into the body by viral vectors such as retroviruses, adenoviruses, and Sendai viruses and by non-viral vectors such as liposomes. Examples of administration methods include *in vivo* and *ex vivo* methods.

Next, the expression level or activity of Int6 in non-human transgenic animals (or tissues or cells derived from these animals) is measured. Alternatively, angiogenic effects in these animals (or tissues derived from these animals) are observed.

Then, compounds that decrease the expression level or activity of Int6 gene, or compounds that suppress angiogenesis compared with when the test substance is not administered (control) are selected.

Pharmaceutical agents of the present invention can be administered orally or parenterally as pharmaceutical compositions by mixing with pharmaceutically acceptable carriers, excipients, diluents, and such. Oral agents can be made into dosage forms such as granules, powders, tablets, capsules, solutions, emulsions, or suspensions. Dosage forms such as injections, drip infusions, topical agents, or suppositories can be selected for parenteral agents. Injections may include subcutaneous injections, intramuscular injections, and intraperitoneal injections. Topical agents may include transnasal agents and ointments. Techniques for formulating these dosage forms so as to include the pharmaceutical agents of the present invention as the main ingredient are well known.

For example, tablets for oral administration can be prepared by adding an excipient, disintegrator, binding agent, lubricant, or such to a pharmaceutical agents of the present invention, mixing them, and then compressing and molding the mixture. As an excipient, lactose, starch, mannitol, or such is generally used. As a disintegrator, calcium carbonate, carboxymethyl cellulose calcium, or such is generally used. As a binding agent, gum arabic, carboxymethyl cellulose, or polyvinylpyrrolidone is generally used. As a lubricant, talc, magnesium stearate, and such, are well known.

Tablets comprising the pharmaceutical agents of the present invention can be coated for masking or to form an enteric-coated formulation using well-known methods. As a coating agent, ethylcellulose, polyoxyethylene glycol, or such can be used.

An injection can be obtained by dissolving a pharmaceutical agent of the present invention, which is a major ingredient, together with an appropriate dispersant, or by dissolving or dispersing in a dispersion medium. Either an aqueous form or an oily form can be prepared for the dosage form by selecting a dispersion medium. To prepare an aqueous form, distilled water, physiological saline, Ringer's solution, or such should be used as a dispersion medium. For an oily form, various plant oils, propylene glycol, and such are used as a dispersion medium. Preservatives such as paraben can be added if necessary. A well-known isotonic agent, such as sodium chloride, glucose, or such, can also be added to the injection. Furthermore, a soothing agent, such as benzalkonium chloride or procaine hydrochloride can also be added.

Furthermore, pharmaceutical agents of the present invention may be used as topical agents when formulated into solid, liquid, or semi-solid compositions. A solid or liquid composition can be made into a topical agent by forming a composition similar to those mentioned previously. A semi-solid composition can be prepared by adding a thickening agent to a suitable solvent as necessary. As solvent, water, ethyl alcohol, polyethylene glycol, or such can be used. As thickening agent, bentonite, polyvinyl alcohol, acrylic acid, methacrylic acid, polyvinylpyrrolidone, or such is generally used. Preservatives such as benzalkonium chloride can be added to this composition. Furthermore, a suppository can be prepared by combining with an oily base such as cacao butter, or an aqueous gel base such as a cellulose derivative as a carrier.

A pharmaceutical agent of the present invention is administered at necessary quantity to mammals including humans within a dose range that is considered safe. Dosage of a pharmaceutical agent of the present invention can be determined appropriately by considering the type of dosage form, method of administration, age and body weight of the patient, symptoms of the patient, and such, and ultimately by the decision of a physician or veterinarian.

"Therapeutic agents" in the present invention include pharmaceutical agents having preventive effects as well as therapeutic effects.

All of the prior-art literature cited herein is incorporated herein by reference.

### Examples

Herein below, the present invention will be specifically described with reference to Examples, but it is not to be construed as being limited thereto.

### [Example 1] Yeast two-hybrid library screening

Clone CL263 was obtained by screening 1.2 x 10⁶ genes using a library derived from human heart or brain (pACT2-cDNA, Takara) by the yeast two-hybrid method (Clontech). The sequencing result identified clone CL263 to be Int6, because the amino acid sequence of the region in clone CL263 that bound to HIF2α matched aa3-aa151 of Int6. The functions of Int6 are mostly unknown. To date, Int6 is known to comprise three parts. Several evidences suggest normal Int6 functions are important for preventing the development of mammary gland tumors in mice and humans.

### [Example 2] Specific interaction of Int6 with HIF1α, HIF2α, and HIF3α in yeast cells

The binding (interaction) activity between Int6 and HIF2α, was assessed by the yeast two-hybrid method. The results are shown in Table 1.

**Table 1**

| pGBKT7 | pACT2 | α-galactosidase activity | β-α-galactosidase activity |
|---|---|---|---|
| HIF1α | Int6 | | |
| HIF2α | Int6 | + | + |
| HIF3α | Int6 | | |
| Vector | Int6 | | |
| HIF1α | Vector | | |
| HIF2α | Vector | | |
| HIF3α | Vector | | |

As a result, only the HIF2α-Int6 combination was found to have α-galactosidase activity and β-α-galactosidase activity, and this revealed that HIF2α and Int6 interact.

### [Example 3] Interaction between HIF2α and Int6 in MCF7, A549, and COS7 cells

Plasmid pMepHA-Int6wt was transfected into three types of cells, MCF7, A549, and COS7. Later, Int6 was found to be expressed mainly in the cytosol of cells. Although confirmation on IF was difficult due to cell death, some Int6 that has transferred to the nucleus was observed to colocalize with HIF2α.

Int6 was found to suppress the expression of HIF2α when cotransfected with HIF2α (Table 2).

**Table 2**

| Plasmid DNA | Treatment | HIF2α expression | | Plasmid DNA | Treatment | HIF2α expression | |
|---|---|---|---|---|---|---|---|
| | | BHK | MCF7 | | | BHK | MCF7 |
| HIF2α | Nor 4H | 5% | 10% | HIF2α + Int6 | Nor 4H | 0.2% | 0.6% |
| | Nor 12H | 2% | 5%-10% | | Nor 12H | 0.2% | 0.5% |
| | Hyp 4H | 7% | 20%-30% | | Hyp 4H | 0.1% | 1% |
| | Hyp 12H | 10% | 20%-30% | | Hyp 12H | 0.2% | 0.5%-1% |
| | LG 5H | 1% | 5% | | LG 5H | 0.1% | 0.1% |
| | LG 20H | 1% | 1% | | LG 20H | 0.1% | 0.05% |

### [Example 4] Regulation of the transcriptional activity of HIF2α by Int6

Experiments were performed with MCF7 cells to assess whether the presence of PINT (PCI) domain in Int6 affects HIF2α transcriptional activity, and whether the transcriptional activity of HIF2α is regulated by Int6. MCF7 cells were cultured under normoxic conditions and hypoxic conditions for four hours or 24 hours, and HIF2α, wild-type Int6 that has the Int6 PINT (PCI) domain (Int6 wt), and a mutant in which Int6 PINT (PCI) domain has been deleted by molecular biological means (Int6-ΔC) were used.

The result revealed that Int6 regulates the HIF2α transcriptional activity through its PINT (PCI) domain (Fig. 2). When coexpressed with HIF2α, Int6-ΔC promoted the transcriptional activity of HIF2α under both normoxic conditions and hypoxic conditions. Int6 wt caused cell death of MCF7 cells, and suppressed the transcriptional activity of HIF2α in both normoxic conditions and hypoxic conditions (Fig. 2).

### [Example 5] Suppression of the expression of the endogenous Int6 gene by Int6 siRNAs

Experiments were performed to assess whether endogenous Int6 is suppressed by siRNAs. siRNA expression plasmids were prepared by using pSilence 2.1-U6 vectors from Ambion, and designing the inserts of hairpin siRNA (Int6 siRNA 145), 2 (Int6 siRNA 219), and 3 (Int6 siRNA 358). Then the siRNA expression plasmids and Int6-ΔC were cotransfected into MCF7 cells.

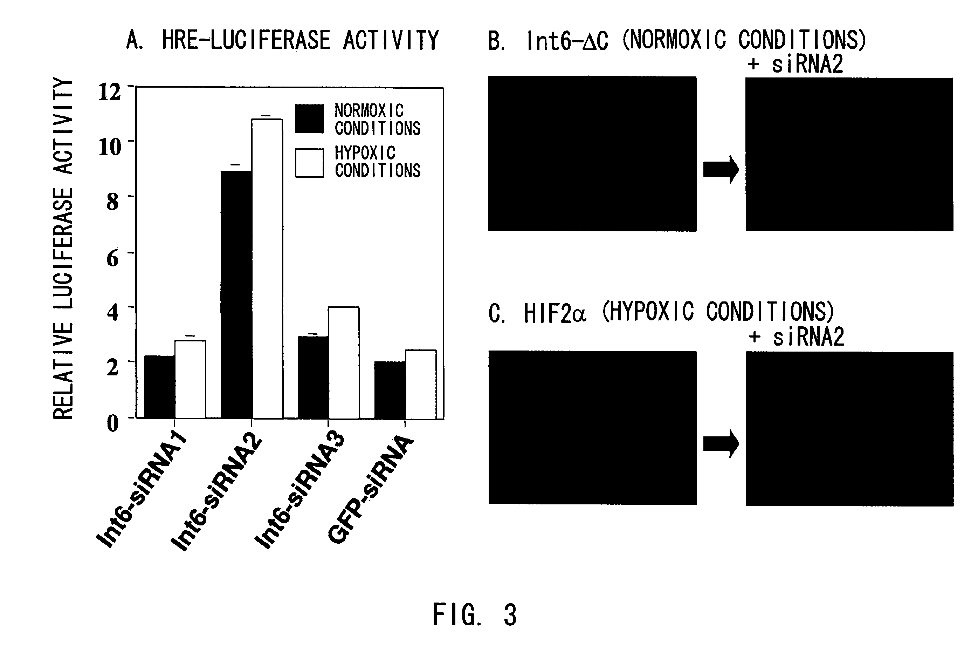

The luciferase activity of hypoxia response elements (HREs) was confirmed under normoxic conditions and hypoxic conditions, and siRNA2 was found to show the greatest suppression of endogenous Int6 under both normoxic conditions and hypoxic conditions (Fig. 3A). While the expression of Int6-ΔC in MCF7 cells under normoxic conditions was confirmed by immunostaining, the expression of Int6-ΔC was not observed due to siRNA2 (Fig. 3B). Additionally, the expression of HIF2α in MCF7 cells under hypoxic conditions was found to increase by siRNA2 (Fig. 3C).

The *in vitro* suppressive effects of the siRNAs were confirmed by the immunostaining method. As a result, the percentage of Int6-ΔC expression was shown to be suppressed with siRNA1, 2, and 3 by 50%, 100%, and 70%, respectively.

Human Int6 oligo sequences that were used are as follows:
1. 5-Int6 RNAi-145: 
gAT CCC / AAC ATg gTA gAC TTT gCT A / TTC AAg AgA / T AgC AAA gTC TAC CAT gTT
/ TTT TTT / ggA AA (SEQ ID NO: 8)
2. 5-Int6 RNAi-219: 
gAT CCC / AAg AAC CAC AgT ggT TgC A / TTC AAg AgA / T gCA ACC ACT gTg gTT CTT
/ TTT TTT / ggA AA (SEQ ID NO: 5)
3. 5-Int6 RNAi-358:

gAT CCC / AAg CAT ggT TTT Agg CAg g / TTC AAg AgA / C CTg CCT AAA ACC ATg CTT
/ TTT TTT / ggA AA (SEQ ID NO: 6)

In each of these nucleotide sequences, the underlines indicate "sense oligos" and "antisense oligos". RNA molecules that form double strands at these regions function as siRNAs.

### [Example 6] Int6 gene expression-knockdown animals generated using siRNAs

### (1) Preparation of HVJ-E encapsulated siRNAs

HVJ-E encapsulated siRNA was prepared for each mouse as follows:
placed 40 µL of 1AU HVJ Envelope (HVJ-E):GenomONE-Neo (Ishihara Sangyo) in a microtube;
centrifuged at 12,000 rpm, 4°C for five minutes to precipitate;
added 10 µL of 0.5 mg/mL pSilencer 1.0V6 mouse Int6-219, or added pSilencer 1.0V6 mouse GAPDH as a control, and mixed them;
added 1 µL reagent B (encapsulating agent), and mixed them;
centrifuged at 12,000 rpm, 4°C for five minutes to precipitate; and
added 40 µL of PBS, and suspended by pipetting 20 to 30 times.

The above was performed according to the specifications of GenomONE-Neo (Ishihara Sangyo).

### (2) Assessment of the effects of the siRNA (Int6) on mouse angiogenesis

Ten-week old female BALB/C mice (body weights of about 25 g) were used for the experiment. Two days before subcutaneous injection with Int6 vector-type siRNA, the cervicodorsal region of the mice was treated with a depilatory cream. On the day of the experiment, the mice were subjected to halothane anesthesia, and 50 µL of HVJ-E-encapsulated siRNA was subcutaneously injected into the cervicodorsal region using a 27GX3/4 syringe. Thereafter, the mice were reared for five days on normal feed and water. Next, the mice were sacrificed by cervical dislocation, and 1.5 cm² or so of skin with the site of siRNA injection in the center was removed. After the back side of the skin was photographed, it was fixed with formalin.

The result from comparison with the control siRNA showed promotion of angiogenesis increased in the order of Int6 siRNA1, Int6 siRNA3, and Int6 siRNA2.

Mouse Int6 oligo sequences that were used are as follows. Each oligo sequence was prepared based on the mouse sequence.
(1) Int6 siRNA1-145:
   5'-AAT ATg gTg gAC TTT gCT A/T-FC AAg AgA/T AgC AAA gTC CAC CAT ATT/TTT TTT (SEQ ID NO: 9)
(2) Int6 siRNAi2-219: 
5'-AAg AAC CAC AgT TgT TgC g / TTC AAg AgA / C gCA ACA ACT gTg gTT CTT / TTT
TTT (SEQ ID NO: 10)
(3) Int6 siRNAi3-358:

5'-AAA CAT ggg TTT Agg CAA g/TTC AAg AgA/C TTg CCT AAA CCC ATg TTT/TTT TTT
(SEQ ID NO: 11)

In each of these nucleotide sequences, the underlines indicate "sense oligos" and "antisense oligos". RNA molecules that form double strands at these regions function as siRNAs.

### [Example 7] Suppression of Int6 mRNA expression by hormone treatment

Estrogen (estradiol, E2), progesterone (Progestin), or the estrogen receptor-suppressing agent tamoxifen (4OH-TAM) was added to breast cancer cell line MCF-7. After culturing for 24 hours, the mRNA of these cells was extracted and the amount of mRNA was quantified by Real Time PCR.

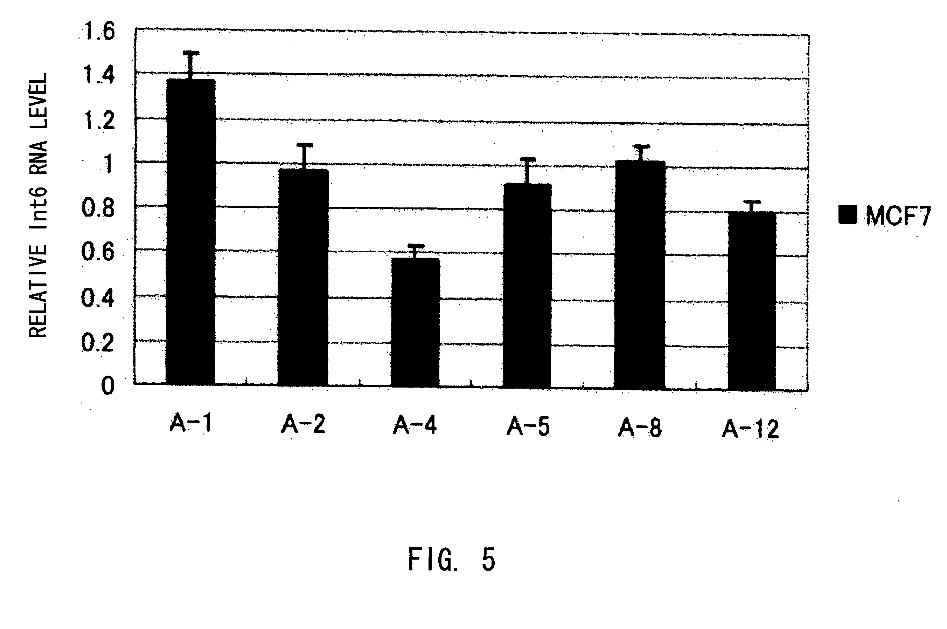

As a result, as shown in Fig. 5, treatment with various kinds of female hormones was found to suppress the expression of Int6 gene. Furthermore, progesterone showed a more obvious suppressive effect than estradiol (E2).

### [Example 8] Regulation mechanism of angiogenesis by Int6 via HIF2α

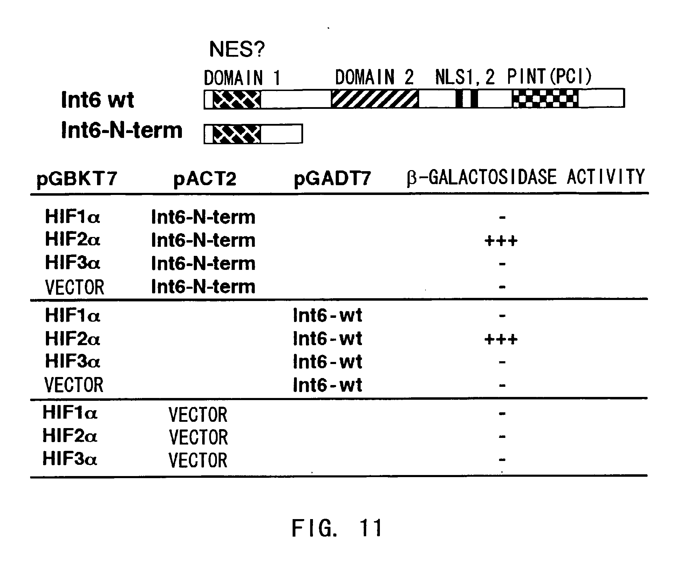

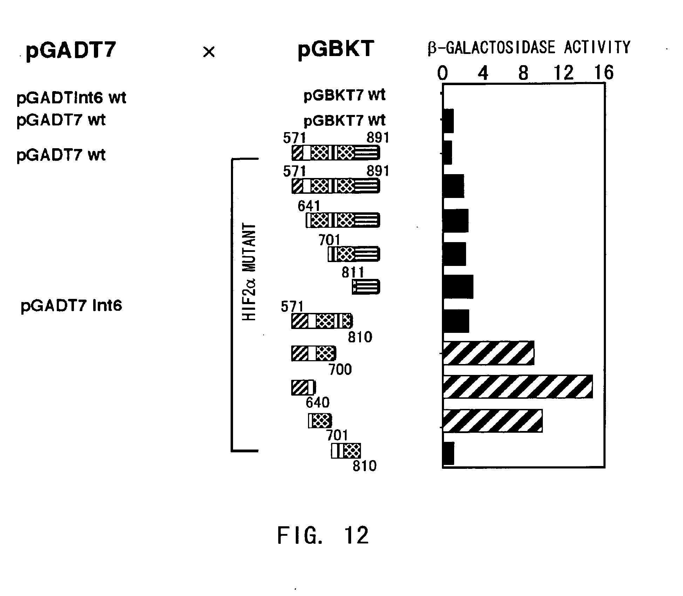

The results of studies using the yeast two-hybrid method and VEGF promoter assay revealed that, similarly to VHL for HIF1α, Int6 interacts directly with HIF2α (Fig. 11), with the binding site at the 571-640 aa region of the HIF2α C terminus (Fig. 12), and strongly suppress the HIF2α-mediated transcription of hypoxic stress-responsive genes.

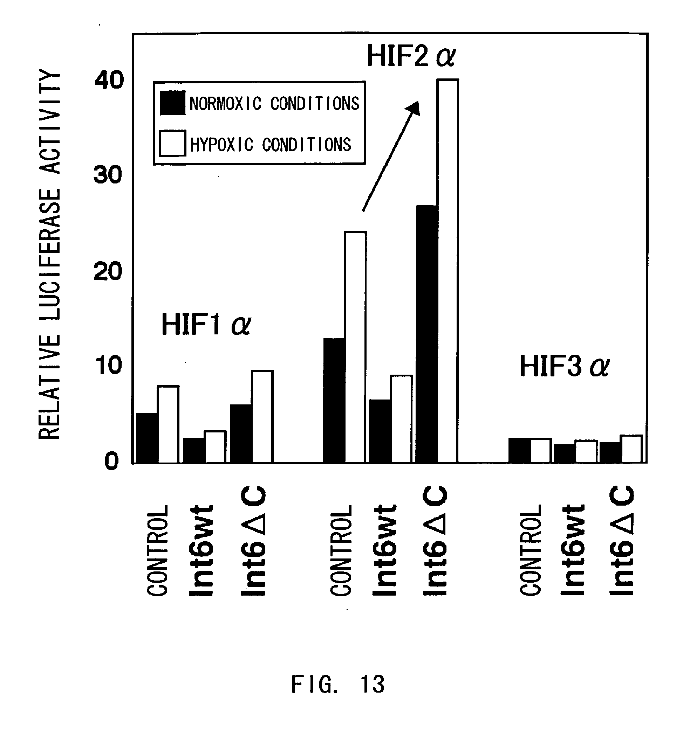

In direct contrast to the wild type, the constitutively inactive (dominant negative) C-terminal deleted mutant produced by MMTV mutation nearly doubled the expression and transcriptional activity of HIF2α (Fig. 13). This result reveals a new mechanism of canceration, in which the Int6 mutation that occurred during the integration of MMTV into genome induces cell growth and angiogenesis as a result of the long term increase in HIF2α activity, leading to canceration.

The essence of HIF2α regulation by Int6 is understood as: Int6 has a region in its C terminus that binds to the proteasome lid (lid portion), and HIF2α bound to Int6 is brought into proteasome for degradation. The hypoxia-independent aspect of HIF2α has been reported, and the present inventors showed that while HIF1α is 100% hypoxia-dependent, the hypoxia-independent degradation of HIF2α is regulated by Int6. More specifically, HIF2α is considered to be regulated mainly by Int6.

Substances that inhibit Int6 gene expression or its function (for example, HIF2α-binding activity) are expected to show angiogenic effects by promoting the expression of HIF2α and transfer of HIF2α to the nucleus independently of hypoxia, to promote the transcription of a group of hypoxic stress-responsive genes. Such substances are expected to have significant angiogenic effects and specificity, and therapeutic effects against various vascular diseases including arteriosclerosis obliterans, restenosis after percutaneous vasodilation treatment (PCT), and myocardial infarction.

### [Example 9] Normal angiogenesis by subcutaneously introducing into mice mouse siRNA against the Int6 gene (Int6-siRNA)

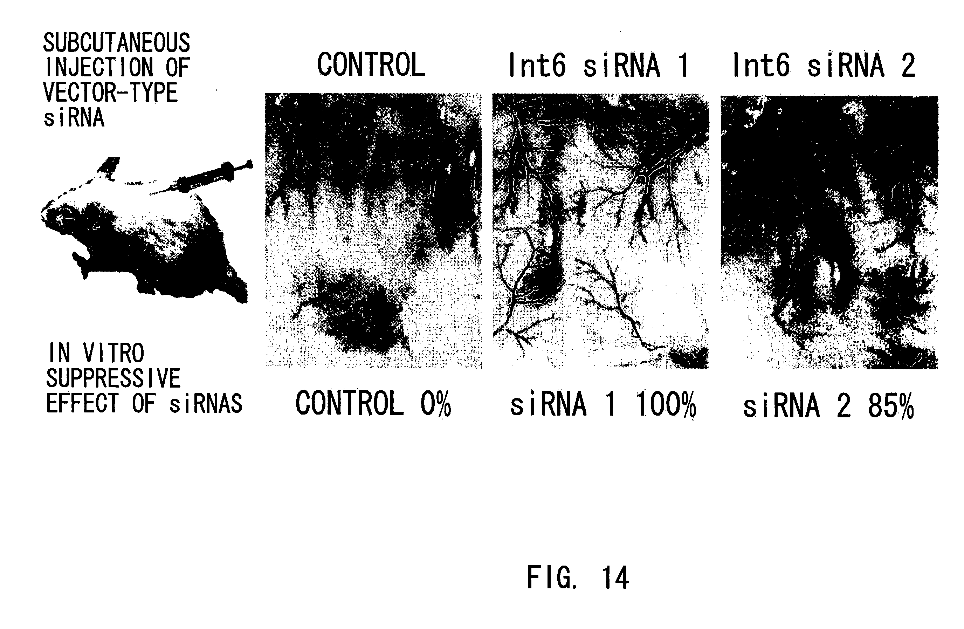

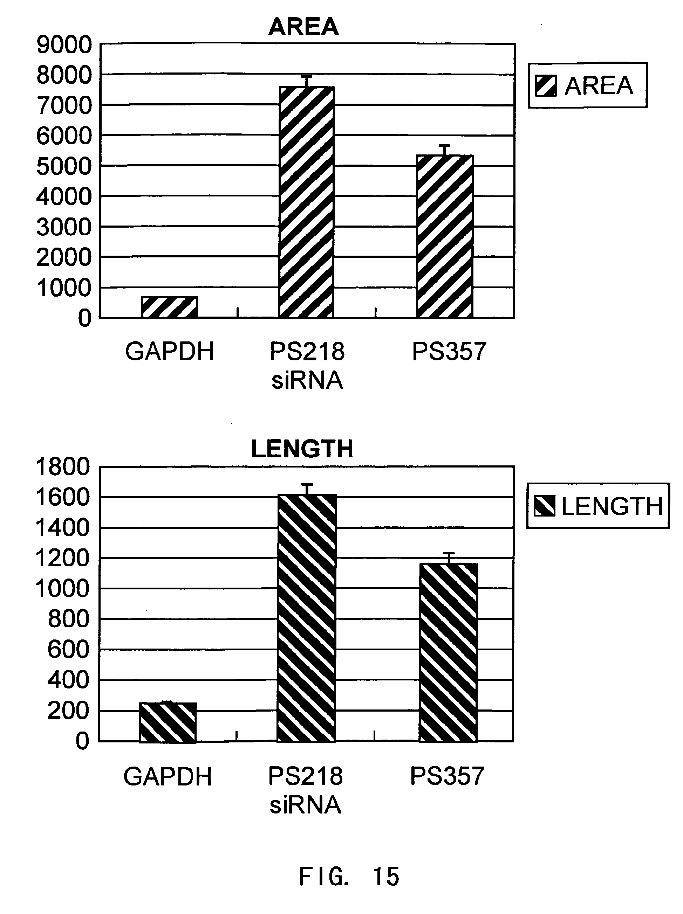

Subcutaneous gene transfer of the siRNA expression vectors into mice using GenomOne (HVJ-envelope gene introducing reagent, Ishihara Sangyo) showed that significant angiogenic effects could be obtained five days after introduction (Fig. 14). An angiogenesis analysis software (Kurabo) was used, and the results showed a significant increase of five to ten folds in the vascular area and vascular length (Fig. 15).

It was observed that these results were proportional to the quantity of vector introduced, and clearly dependent on the effects of the expressed siRNAs. In addition, results from a detailed analysis using an *in vitro* angiogenesis analysis kit (Kurabo) confirmed that the effects of the synthetic siRNAs were also quantity-dependent. The angiogenic effects due to HIF2α overexpression were also positive, strongly suggesting that Int6-siRNA induced the expression of endogenous HIF2α, which induced a group of angiogenic factors.

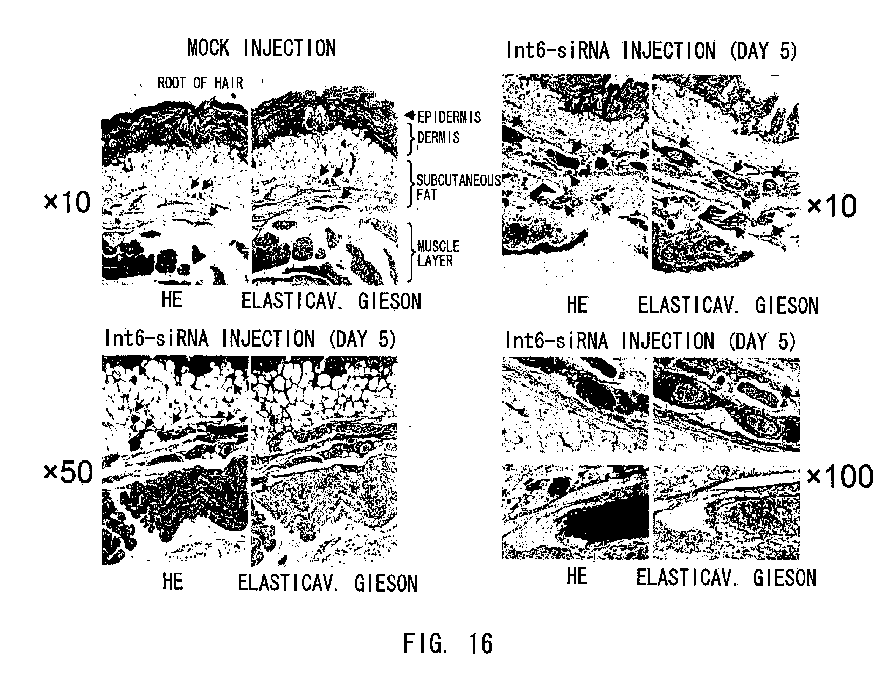

Pathological analyses showed that newly formed blood vessels were normal arteries with endothelia and elastic fibers, and ecchymoses found in neoplastic angiogenesis were absent. Furthermore, the Int6 siRNA expression vectors were mainly introduced into vascular endothelial fibroblasts, and this suggested that a group of angiogenic factors are secreted from these fibroblasts (Fig. 16).

### [Example 11] Confirmation that canceration does not occur by the introduction of Int6-siRNA into cells

Int6 was found to act as a tumor suppressor factor during canceration in breast cancer and such. Accordingly, there was a concern that canceration would occur when specific siRNA were introduced into cells, and this might become a significant problem particularly in clinical applications. To examine this problem in detail, the present inventors introduced into cells a vector-type siRNA that constitutively expresses Int6-siRNA when introduced into cells, and observed for a long period of time. In cancerous cell lines such as MCF-7 and HeLa cells, introduction of a vector-type siRNA that completely suppresses endogenous Int6 shortened the survival of cells, cell growth stopped within seven weeks, and cell death was induced. This can be interpreted to mean that one of the roles of Int6 is translational control, and its complete suppression causes abnormalities in translational control.

In experiments using primary culture fibroblasts obtained from mice and experiments using cultured cells for angiogenesis analysis from Kurabo, the results from eleven days of observation indicated that while introduction of siRNA expression vectors and synthetic siRNAs showed effective angiogenesis, canceration was not observed at all.

### Industrial Applicability

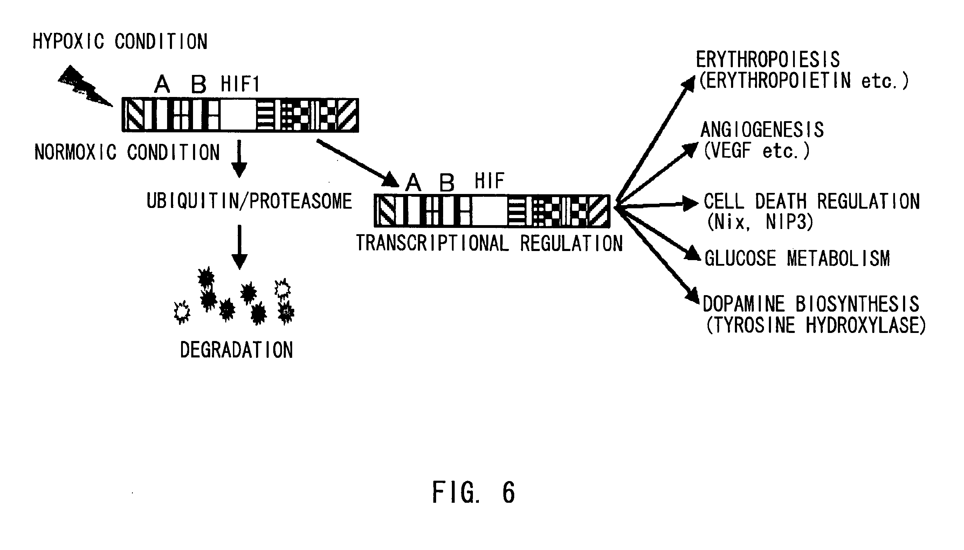

Of the genes involved in hypoxic stress response, the transcription factors HIF (hypoxia-inducible factors) are reported. These factors are known to produce vascular endothelial growth factors (VEGFs) which are necessary for tumor angiogenesis, and to regulate the transcription of many glycolytic pathway enzymes involved in the energy metabolism of mitochondria. In solid tumors (for example, cancers), the condition becomes fairly hypoxic inside of the tumor in spite of abundant blood flow, and this condition consequently promotes angiogenesis. Besides angiogenesis, HIFs take on various roles in cells, and for example, enhancement of erythropoietin gene expression is necessary for erythrocyte growth under hypoxia, and increased VEGF gene expression is important for angiogenesis (Fig. 6).

At normal oxygen concentration (21 %), HIFα is degraded by the ubiquitin-proteasome system, but under hypoxic conditions, it escapes this degradation and transfers into the nucleus to evoke induction of various factors as a transcription factor. HIFs themselves are mainly classified into three types, HIF1α, HIF2α, and HIF3α. In contrast to HIF1α which is expressed ubiquitously, HIF2α is expressed specifically in the vascular endothelium, glia of the brain, fibroblasts, skeletal muscles, and cardiac muscles; and HIF3α is expressed specifically in the cerebral nervous system. HIF1α is known to be related to ischemic diseases such as cerebral infarction and myocardial infarction. HIF2α is involved in angiogenesis in hypoxic stress-related diseases, particularly cancers. The behavior of HIF1α inside cells is being elucidated. In contrast, although several factors that bind to HIF2α have been reported, factors that regulate its function have not been elucidated at all. From the deep involvement of HIF2α in angiogenesis, it was predicted that regulating its intracellular behavior would allow new induction of angiogenesis, and thus the present inventors analyzed novel regulatory factors of HIF2α.

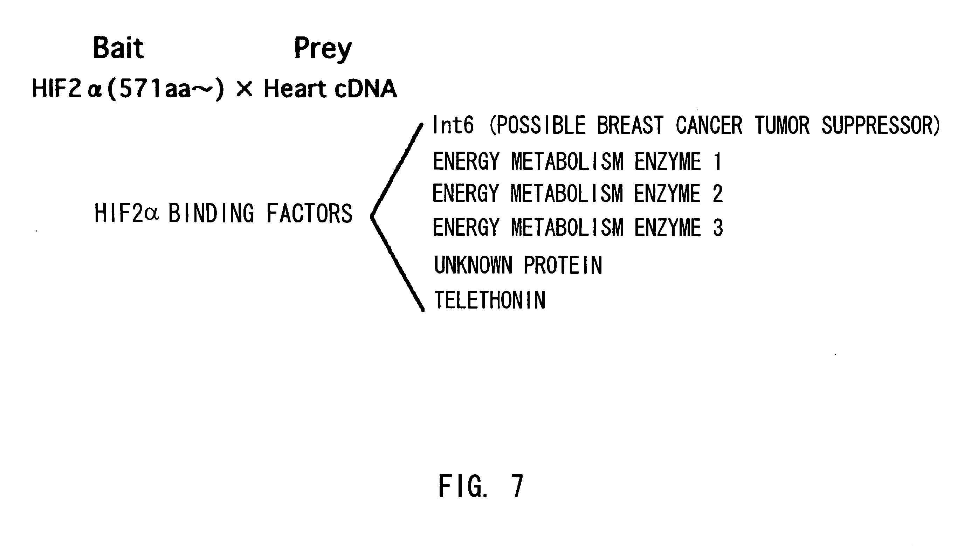

The present inventors successfully identified from a human heart or brain library, six new factors as HIF2α-interacting factors using the yeast two-hybrid method (Fig. 7). Of these factors, Int6 is reported to be a tumor inhibitor for breast cancer in mice and humans, but its action mechanism is completely unknown.

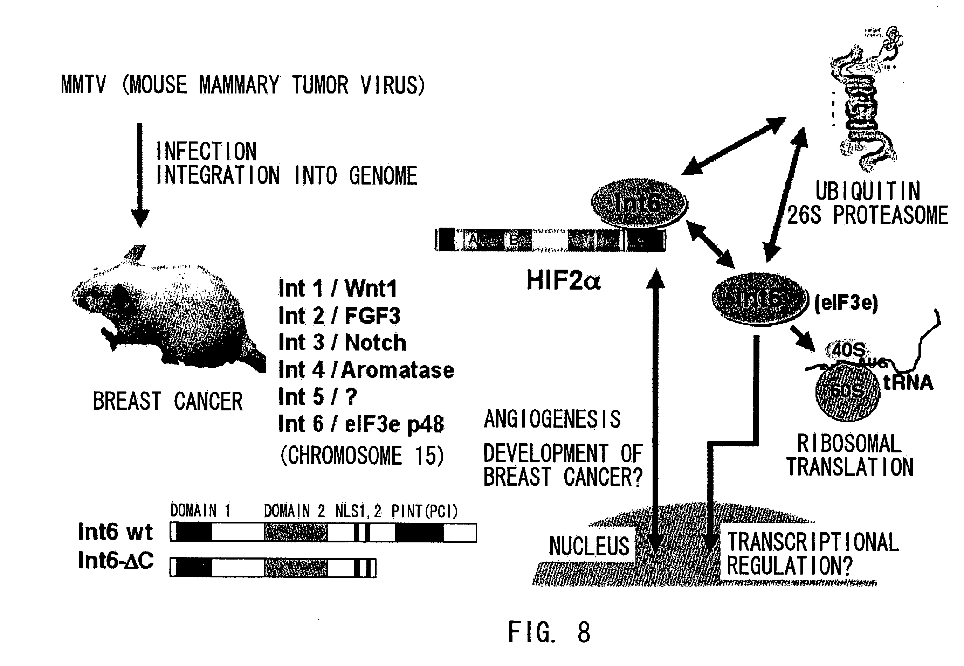

Int6 is originally a translation regulatory factor eIF3e/p48, and when a mouse mammalian tumor virus (MMTV) infects mice, it is integrated at nine gene loci and targeted genes at those sites are disrupted. Since it corresponds to the sixth integration site in the disrupted gene, it was named Int6. Infection of MMTV caused Int6 to form a constitutively inactive (dominant negative) mutant (Int6-ΔC) missing a part of the C terminus, and breast cancer developed (Fig. 8). However, the mechanism as to how this mutant develops breast cancer is unclear.

The present inventors identified the Int6 protein as a factor that binds to HIF2α, and revealed for the first time that substances inhibiting Int6 expression or its function, and dominant negative mutants of Int6 (Int6-ΔC) can increase angiogenic effects by enhancing the activity of HIF2α to promote transcription of hypoxic stress-responsive genes.

In particular, substances that have effects of suppressing Int6 expression are expected to have significant angiogenic effects, and are expected to be applied to treatment of arteriosclerosis obliterans, restenosis after percutaneous vasodilation treatment (PCT), myocardial infarction, and such. Specifically, siRNAs that are expected to have specific suppressive effects on Int6 expression promote the expression of therapeutically effective genes by suppressing the expression of transcription regulatory factors, and are highly expected to be an entirely novel-concept therapeutic agent.

Furthermore, by using an siRNA expression vector to knock down Int6, which is conventionally reported to be a translation regulatory factor (eIF3e) or a tumor inhibitor in breast cancer, the present inventors showed that new formation of normal arteries is possible without any canceration in animal experiments using mice. Furthermore, the present inventors showed that the target cells of siRNA are carrier cells such as fibroblasts. This strongly suggests the possibility that new arteries may be formed by culturing and growing carrier cells such as fibroblasts collected from patients, introducing synthetic siRNA *ex vivo,* and then autotransplanting them into their affected sites. The autotransplantation method which returns cells collected from patients after siRNA introduction (siRNA-treated angiogenesis-inducing cells) to their affected sites may be used to avoid the development of carriers, which is a bottleneck in siRNA drug development.

Development of therapeutic methods is anticipated for diseases such as arteriosclerosis obliterans, myocardial infarction, and cerebral infarction, and the number of patients is expected to rise in the future as lifestyle-related diseases increase. These diseases are expected to be cured by constructing new blood vessels. It is expected that construction of new blood vessels can also be applied to resume blood flow to hepatic parenchymal cells in liver cirrhosis, and to regenerate muscles from vascular development in neuromuscular degenerative diseases. Also in regenerative medicine that uses embryonic stem cells (ES cells) and stromal stem cells, angiogenesis is necessary for securing blood circulation to reconstructed tissues.

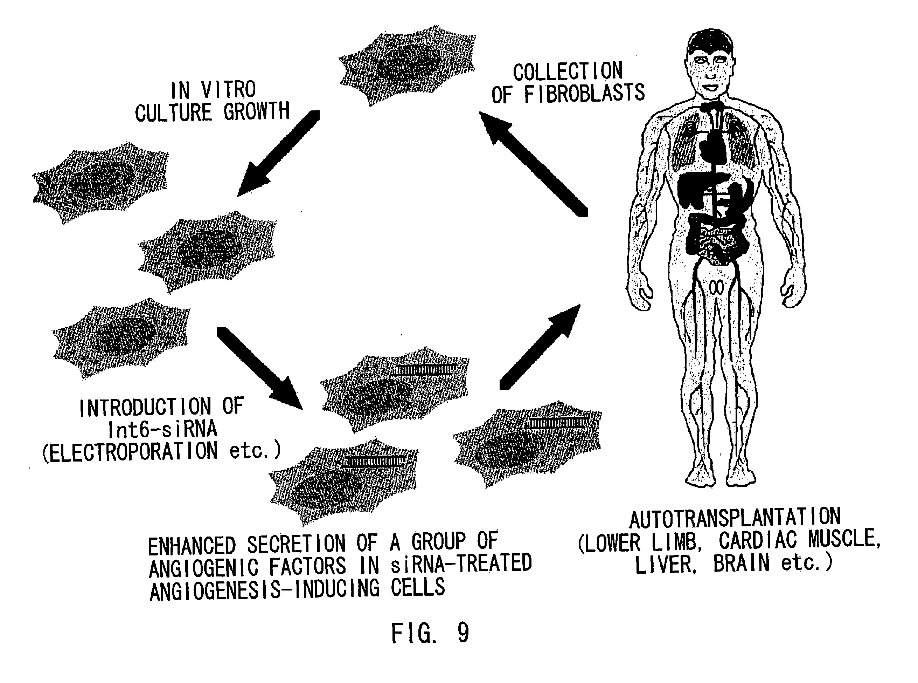

The present invention provides neovascular bypass therapy that can be applied to treatment of arteriosclerosis obliterans, myocardial infarction and cerebral infarction, and regenerative medicine such as hepatic regeneration, where carrier cells such as patients' fibroblasts are autotransplanted after being treated with synthetic siRNA against Int6 (siRNA-treated angiogenesis-inducing cells). As an example of neovascular bypass therapy of the present invention, the basic concept of this method is depicted in Fig. 9.

As shown in the Examples of the present application, the present inventors demonstrated that substances which inhibit Int6 expression or its function effectively increase arterial angiogenesis and confirmed this at the level of animal experiments. Notably, siRNAs that are expected to have specific suppressive effects on Int6 gene expression promote the expression of genes that have therapeutic effects for vascular diseases by suppressing the expression of transcription regulatory factors, and are highly expected to be an entirely novel-concept therapeutic agent for vascular diseases. In conventional gene therapy that aims at angiogenesis, only the genes of interest are forcibly expressed. In contrast, siRNA agents based on this concept (angiogenesis-inducing siRNA agents) can increase the overall expression of a group of genes of interest that are necessary for angiogenesis by specifically suppressing the expression of target genes, and thus they are expected to yield significant therapeutic effects.

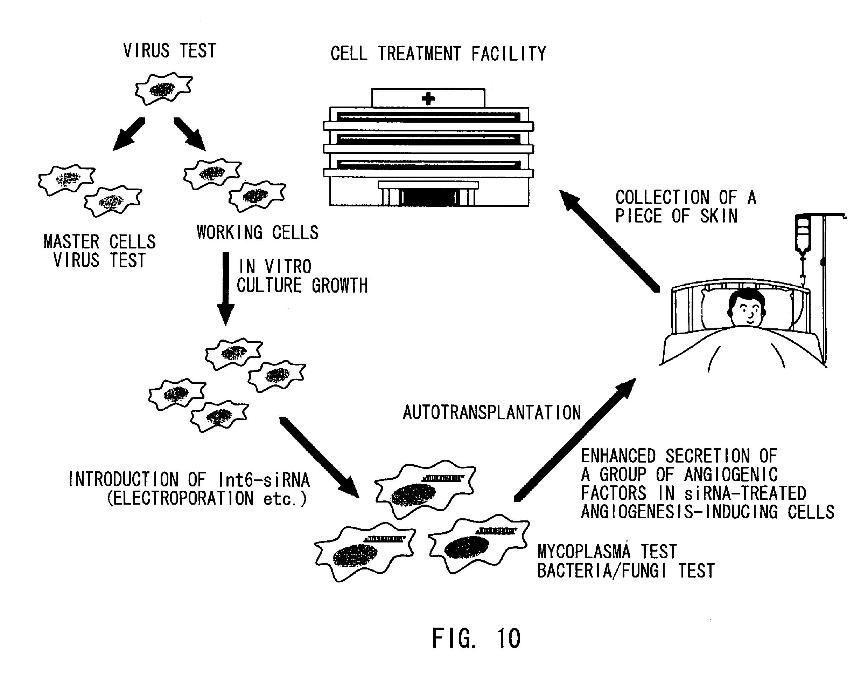

Detailed assessment of the results from the animal experiments of the present invention suggested the possibility that angiogenesis-inducing siRNA expresses effects in fibroblasts. This indicates a possible utility of "siRNA-treated angiogenesis-inducing cells" based on the completely novel concept, in which specific suppression of Int6 gene expression in fibroblasts causes comprehensive expression of a group of hypoxic stress-responsive genes that are effective for angiogenesis (Fig. 10). Since fibroblasts can be obtained from patients themselves, grown, and then easily autotransplanted, the probability of rejection reactions and such when transplanting the "siRNA-treated angiogenesis-inducing cells" is predicted to be low.

Regarding the autotransplantation of carrier cells such as fibroblasts, its use as artificial skin has already been demonstrated, and it may be possible to prepare "siRNA-treated angiogenesis-inducing cells" following those protocols. Specifically, carrier cells such as fibroblasts are collected from a piece of skin from patients that have a negative virus test result, and mass cultured to prepare master cells and working cells. After the master cells were re-confirmed to be negative on the virus test, the working cells were sequentially thawed and passaged to prepare carrier cells for "siRNA-treated angiogenesis-inducing cells". When preparing the cells, it is necessary to perform mycoplasma test and bacteria/fungi tests to confirm that the results are negative. The carrier cells prepared for "siRNA-treated angiogenesis-inducing cells" were stored by freezing, and may be provided when necessary.

Furthermore, high introduction efficiency can be achieved by introducing angiogenesis-inducing siRNA into patient-derived fibroblasts *via* an *ex vivo* electroporation method or such. Since this method transiently forms "siRNA-treated angiogenesis-inducing cells" using siRNA, and the possibility of cell canceration and such due to genetic modification is low, it can be regarded as an exceedingly safe method compared with the method of preparing cells for transplantation using a gene expression vector of interest (gene therapy). At the same time, this does not require a special delivery system which is considered a problem in conventional siRNA drug development.

Furthermore, for the "siRNA-treated angiogenesis-inducing cells" of the present invention, carrier cells are easily collected from patients and grown. It is expected that in transplantation of myeloid mononuclear cells, it would be difficult to exert the effects in parts where the blood flow is significantly stagnant, because vascular endothelial precursor cells, which mainly exhibit angiogenic effects, are provided through blood flow. In contrast, "siRNA-treated angiogenesis-inducing cells" in this study of practical application are expected to be effective even in regions where the blood flow is stagnant because they can be transplanted to any site by direct injection. Additionally, regions of stagnant blood flow are under hypoxic condition and this hypoxic condition is expected to further increase the activity of endogenous HIF2α in "siRNA-treated angiogenesis-inducing cells", thereby yielding a synergistic effect.

The present invention revealed Int6 will be a suitable target molecule for drug development. The various findings made in the present invention are also very useful academic information for elucidating the mechanisms of hypoxic stress response.

## Claims

1. A hypoxic stress response-promoting agent comprising as an active ingredient a substance that suppresses Int6 protein expression or a substance that suppresses Int6 protein function.

2. The hypoxic stress response-promoting agent of claim 1, wherein the substance that suppresses Int6 protein expression is a compound selected from the group consisting of:
(a) an antisense nucleic acid against an Int6 gene transcript or a part thereof;
(b) a nucleic acid having a ribozyme activity of specifically cleaving an Int6 gene transcript; and
(c) a nucleic acid having a function of inhibiting Int6 gene expression by RNAi effect.

3. The hypoxic stress response-promoting agent of claim 1, wherein the substance that suppresses Int6 protein expression is a female hormone, a female hormone secretion-promoting agent, or a receptor agonist of female hormone.

4. The hypoxic stress response-promoting agent of claim 1, wherein the substance that suppresses Int6 protein function is a compound selected from the group consisting of:
(a) an antibody that binds to an Int6 protein;
(b) a low-molecular-weight compound that binds to an Int6 protein; and
(c) a compound that inhibits the binding activity between Int6 protein and HIF2α protein.

5. The hypoxic stress response-promoting agent of any one of claims 1 to 4, wherein the promotion of hypoxic stress response is induction of angiogenesis.

6. A hypoxic stress response-suppressing agent, which comprises as an active ingredient a substance that enhances Int6 protein expression or a substance that enhances Int6 protein function.

7. The hypoxic stress response-suppressing agent of claim 6, wherein the substance that enhances Int6 protein expression is a female hormone-suppressing agent.

8. The hypoxic stress response-suppressing agent of claim 7, wherein the suppression of hypoxic stress response is suppression of angiogenesis.

9. A therapeutic agent for a vascular disease, which comprises the hypoxic stress response-promoting agent of any one of claims 1 to 5 as an active ingredient.

10. The therapeutic agent for a vascular disease of claim 9, wherein the vascular disease is an ischemic brain or heart disease, a neurodegenerative disease, a traumatic brain injury, or a hepatic, pancreatic, muscular, or skin disease.

11. A therapeutic agent for an angiogenesis-related disease, which comprises the hypoxic stress response-suppressing agent of any one of claims 6 to 8 as an active ingredient.

12. The therapeutic agent for an angiogenesis-related disease of claim 11, wherein the angiogenesis-related disease is a cancer disease.

13. A non-human transgenic animal whose Int6 gene expression is artificially suppressed.

14. The non-human transgenic animal of claim 13, wherein the Int6 gene expression is suppressed by the action of any one of the nucleic acids of:
(a) an antisense nucleic acid against an Int6 gene transcript or a part thereof;
(b) a nucleic acid having a ribozyme activity of specifically cleaving an Int6 gene transcript; and
(c) a nucleic acid having a function of inhibiting Int6 gene expression by RNAi effect.

15. The non-human transgenic animal of claim 13 or 14, wherein angiogenic effect is promoted.

16. A method of screening for a therapeutic agent for vascular disease, wherein a compound that decreases the level of Int6 gene expression or Int6 protein activity is selected.

17. A method of screening for a therapeutic agent for vascular disease, which comprises the steps of:
(a) contacting a test compound with Int6 gene-expressing cells;
(b) measuring the level of Int6 gene expression in said cells; and
(c) selecting a compound that decreases the expression level compared with that measured in the absence of the test compound.

18. A method of screening for a therapeutic agent for vascular disease, which comprises the steps of:
(a) contacting a test compound with cells or an extract solution of cells comprising a DNA which comprises a structure in which a reporter gene is operably linked to the transcriptional regulatory region of an Int6 gene;
(b) measuring the expression level of said reporter gene; and
(c) selecting a compound that decreases the expression level compared with that measured in the absence of the test compound.

19. A method of screening for a therapeutic agent for vascular disease, which comprises the steps of:
(a) contacting a test compound with an Int6 protein, or cells or an extract solution of cells expressing the protein;
(b) measuring the activity of said protein; and
(c) selecting a compound that decreases the activity of said protein compared with that measured in the absence of the test compound.

20. A method of screening for a therapeutic agent for vascular disease, which comprises selecting a compound that suppresses the binding activity between Int6 protein and HIF2α protein.

21. A method of screening for a therapeutic agent for vascular disease, which comprises the steps of:
(a) contacting a test compound with an Int6 protein and an HIF2α protein;
(b) measuring the binding activity between the Int6 protein and the HIF2α protein; and
(c) selecting a compound that decreases said binding activity compared with that measured in the absence of the test compound.

22. A method of screening for a therapeutic agent for angiogenesis-related disease, which comprises selecting a compound that increases the level of Int6 gene expression or Int6 protein activity.

23. A method of screening for a therapeutic agent for angiogenesis-related disease, which comprises the steps of:
(a) contacting a test compound with Int6 gene-expressing cells;
(b) measuring the level of Int6 gene expression in said cells; and
(c) selecting a compound that increases the expression level compared with that measured in the absence of the test compound.

24. A method of screening for a therapeutic agent for angiogenesis-related disease, which comprises the steps of
(a) contacting a test compound with cells or an extract solution of cells comprising a DNA which comprises a structure in which a reporter gene is operably linked to the transcriptional regulatory region of an Int6 gene;
(b) measuring the expression level of said reporter gene; and
(c) selecting a compound that increases the expression level compared with that measured in the absence of the test compound.

25. A method of screening for a therapeutic agent for angiogenesis-related disease, which comprises the steps of:
(a) contacting a test compound with an Int6 protein, or cells or an extract solution of cells expressing the protein;
(b) measuring the activity of said protein; and
(c) selecting a compound that increases the activity of said protein compared with that measured in the absence of the test compound.

26. A method of screening for a therapeutic agent for angiogenesis-related disease, which comprises selecting a compound that enhances the binding activity between Int6 protein and HIF2α protein.

27. A method of screening for a therapeutic agent for angiogenesis-related disease, which comprises the steps of:
(a) contacting a test compound with an Int6 protein and an HIF2α protein;
(b) measuring the binding activity between the Int6 protein and the HIF2α protein; and
(c) selecting a compound that increases said binding activity compared with that measured in the absence of the test compound.

28. A method of screening for a therapeutic agent for angiogenesis-related disease, which comprises the steps of:
(a) administering a test compound to the non-human transgenic animal of any one of claims 13 to 15;
(b) measuring the level of Int6 expression or activity in said non-human transgenic animal, or the angiogenic effect in said animal; and
(c) selecting a compound that decreases the angiogenic effect, or a compound that increases the level of Int6 expression or activity compared with that when the test compound is not administered.

29. An angiogenesis-inducing agent comprising a dominant negative Int6 mutant as an active ingredient.

30. The angiogenesis-inducing agent of claim 29, wherein the dominant negative Int6 mutant is an Int6 protein mutant in which the C-terminal PINT region of the Int6 protein is deleted.

31. An angiogenesis-suppressing agent comprising an Int6 protein or an Int6 protein expressing-vector as an active ingredient.

32. A carrier cell, wherein Int6 gene expression is suppressed and angiogenesis is induced.

33. The carrier cell of claim 32, into which an siRNA against an Int6 gene has been introduced.

34. A therapeutic agent for vascular disease, which comprises the angiogenesis-suppressing agent of claim 31 or the carrier cell of claim 32 or 33 as an active ingredient.

35. A method for producing a therapeutic agent for vascular disease, which comprises the step of introducing an siRNA against an Int6 gene into isolated carrier cells.
